# EUROPEAN PATENT SPECIFICATION

(11) **EP 3 076 858 B1**
(45) Date of publication and mention of the grant of the patent: **09.09.2020**
(21) Application number: 14824214.2
(22) Date of filing: 05.12.2014
(51) Int. Cl.: A61B 5/00, A61B 5/11

(54) **MOTION-BASED SEIZURE DETECTION SYSTEMS AND METHODS**
BEWEGUNGSBASIERTE ANFALLSERKENNUNGSSYSTEME UND VERFAHREN
SYSTÈMES ET MÉTHODES DE DÉTECTION DE CRISE D'ÉPILEPSIE BASÉS SUR LE MOUVEMENT

(30) Priority: 05.12.2013 US 201361912502 P; 06.12.2013 US 201361913207 P
(43) Date of publication of application: 12.10.2016
(73) Proprietor: Cyberonics, Inc., Houston, TX 77058 (US)
(72) Inventor: SABESAN, Shivkumar, Houston, Texas 77058 (US)
(74) Representative: Grünecker Patent- und Rechtsanwälte PartG mbB
(86) International application number: PCT/US2014/068943
(87) International publication number: WO 2015/085264

(56) References cited:
- WO-A2-2012/125425
- US-A1- 2013 154 827
- JALLON P ET AL: "Detection system of motor epileptic seizures through motion analysis with 3D accelerometers", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3 September 2009 (2009-09-03), pages 2466-2469, XP031882519, DOI: 10.1109/IEMBS.2009.5334770 ISBN: 978-1-4244-3296-7
- NIJSEN T M E ET AL: "Detection of Subtle Nocturnal Motor Activity From 3-D Accelerometry Recordings in Epilepsy Patients", IEEE TRANSACTIONS ON BIOMEDICAL ENGINEERING, IEEE SERVICE CENTER, PISCATAWAY, NJ, USA, vol. 54, no. 11, 1 November 2007 (2007-11-01), pages 2073-2081, XP011194590, ISSN: 0018-9294, DOI: 10.1109/TBME.2007.895114

## Description

### FIELD OF THE DISCLOSURE

The present disclosure relates generally to the monitoring of subject motion and the processing of motion data. More specifically, the present disclosure relates to the processing of subject motion data to monitor subjects susceptible to epileptic events and to detect the occurrence of seizures.

### BACKGROUND

Epilepsy may be characterized by episodes of disturbed brain activity that cause changes in attention or behavior. Increased heart rate, changes in electrocardiogram (ECG) data, changes in electroencephalography (EEG) data, and changes in movement may be correlated to an onset or an occurrence of a seizure. Information can be obtained from an EEG and other sources to characterize and measure the electrical activity of a subject's brain, and the information can be further analyzed to detect the occurrence of a seizure. Likewise, information can be obtained from an ECG, a heart rate monitor, and other sources to characterize and measure electrical activity of a subject's heart, and the information can be further analyzed to detect the occurrence of a seizure.

Seizure-related motion can be exhibited in a variety of body motions, ranging from an episode of no motion or minimal motion to an episode of severe shaking or other extreme movements. Examining motion to detect seizure-related motions is difficult because normal body motions include many motions that mimic or appear similar to seizures. The effective management of epilepsy often necessitates reliable long-term monitoring of seizures, usually over days and months. Although, visual inspection of EEG signals is the current gold standard for seizure detection in supervised environments such as an epilepsy monitoring unit or an intensive care unit where the subject is mostly stationary, it is not practical to use this approach to objectively quantify long-term seizure frequency, especially when the subject is mobile. A current approach to track long-term seizure frequency is by maintaining seizure diaries. However, it has been shown that self-reporting of seizure incidence is severely inaccurate. In this context, seizure detection via the detection of autonomic signatures, such as cardiac or motor signals that are altered by seizures, presents itself as a viable alternative for long-term monitoring of seizures. This approach becomes even more attractive for monitoring the pediatric epilepsy population, especially during the night when supervision is reduced and the risk of SUDEP

(Sudden Unexplained Death in Epilepsy Patients) is high. Wearable devices to chronically monitor cardiac or motor signals associated with seizures can be implemented with greater ease than EEG-based devices and can significantly improve the overall quality of life of patients and caregivers as well as provide an objective way for physicians to track their patients' seizures. Seizures that express themselves in movements or seizures that disturb normal movement patterns can be detected. However, with motion-based seizure detection, motions at any point on the body can influence the motions detected at the point of measurement, and a multitude of normal motions can provide data that overlaps or obscures motion data that can be attributed to a seizure.

Accordingly, what are needed are methods and systems that provide improved measurements of body motion that facilitate the detection of seizures, and that provide and implement improved motion data processing techniques that can be used to identify seizure-related motions within motion data sets containing a multitude of normal motions. Also needed are methods and systems that provide improved resolution of subject motion data to distinguish seizure-related motion from normal motion so as to minimize false positives that may wrongly report the occurrence of a seizure. It is believed that an improved detection of seizure events with motion data and an improved processing of motion data to identify seizures and eliminate or reduce false positives will assist in the diagnosis and treatment of motion-affecting disease states, such as epilepsy, help persons suffering from epilepsy better manage their lives, and assist caregivers in the monitoring of people susceptible to seizures. WO2012125425 defines a device for detecting body orientation and also epileptic seizures.

### SUMMARY

To address these and other unmet needs, the present disclosure provides, in exemplary non-limiting embodiments, systems, devices, and methods for effective seizure detection via the detection of the motion of a subject. In particular, the present disclosure is directed to, among other things, the use of a motion monitoring device to assess motion parameters selected to distinguish between seizure and non-seizure motions.

In at least one embodiment, described further below, a method of distinguishing between a first type of motion and a second type of motion of a subject is disclosed. The first and second types of motion may be characterized by a signal corresponding to the first and second types of motions. The method may include the step of receiving the signal at a processor with the signal being representative of subject motion data of the subject and with the subject motion data including subject position data and subject change-in-position data, and using the processor to analyze the subject motion data to distinguish between the first type of motion occurring over a first time period and the second type of motion occurring over a second time period. The method may characterize the first type of motion as having a first bandwidth that is inclusively within a first bandwidth range, as including subject position data that indicates that the subject is in a recumbent orientation throughout the first time period with the recumbent orientation defined by an initial calibration during which the subject is in the recumbent position while defining an offset angle between a subject axis extending from the subject and a vertical axis and with the recumbent orientation further defined by the subject axis remaining inclusively within an offset angle range throughout the first time period, and as including subject change-in-position data that indicates that a first rotation parameter of the subject change-in-position data is inclusively within a rotation range throughout the first time period. The method may also characterize the second type of motion as having a second bandwidth that is inclusively within a second bandwidth range, as including subject position data that indicates that the subject is in an upright orientation throughout the second time period with the upright orientation defined by the offset angle equaling or exceeding an offset angle threshold throughout the second time period, and as including subject change-in-position data that indicates that a second rotation parameter of the subject change-in-position data is greater than a rotation threshold throughout the second time period. The method may further include the generating of a first output from the processor in response to an identification of the first type of motion and the generating a second output from the processor in response to an identification of the second type of motion.

In at least another embodiment, described further below, a method of detecting a neurological condition of a subject is disclosed. The method may include receiving a signal from the subject at a processor with the signal being representative of subject motion data of the subject and with the subject motion data including subject position data and subject change-in-position data, and may include using a processor to analyze the subject motion data to identify a seizure motion occurring over a first time period and a non-seizure motion occurring over a different second time period. The method may characterize the seizure motion as having a first bandwidth that is inclusively within a first bandwidth range, as including subject position data that indicates that the subject is in a recumbent orientation for at least a portion of the first time period with the recumbent orientation defined by an initial calibration during which the subject is in the recumbent position while defining an offset angle between a subject axis extending from the subject and a vertical axis and with the recumbent orientation further defined by the subject axis remaining inclusively within an offset angle range for at least a portion of the first time period and with subject change-in-position data indicating that a first rotation parameter of the subject change-in-position data is inclusively within a rotation range for at least a portion of the first time period. The method may also characterize the non-seizure motion as having a second bandwidth that is inclusively within a second bandwidth range, as including subject position data indicating that the subject is in an upright orientation for at least a portion of the second time period with the upright orientation defined by the offset angle equaling or exceeding an offset angle threshold for at least a portion of the second time period, and as including subject change-in-position data indicating that a second rotation parameter of the subject change-in-position data is greater than a rotation threshold for at least a portion of the second time period. The method may further include generating a first output from the processor in response to an identification of the seizure motion and generating a second output from the processor in response to an identification of the non-seizure motion.

In yet another embodiment, described further below, a motion monitoring system for monitoring a motion of a subject is disclosed. The motion monitoring system may include a housing, a mounting system configured to couple the housing to the subject, an accelerometer disposed on the housing with the accelerometer configured to obtain subject motion data and with the subject motion data including subject position data and subject change-in-position data, and a processor configured to analyze the subject motion data to distinguish between a first type of motion occurring over a first time period and a second type of motion occurring over a second time period. The motion monitoring system may characterize the first type of motion as having a first bandwidth that is inclusively within a first bandwidth range, as including subject position data indicating that the subject is in a recumbent orientation throughout the first time period with the recumbent orientation defined by an initial calibration during which the subject is in the recumbent position while defining an offset angle between a subject axis extending from the subject and a vertical axis and with the recumbent orientation further defined by the subject axis remaining inclusively within an offset angle range throughout the first time period, and as including subject change-in-position data indicating that a first rotation parameter of the subject change-in-position data is inclusively within a rotation range throughout the first time period. The motion monitoring system may further characterize the second type of motion as having a second bandwidth that is inclusively within a second bandwidth range, as including subject position data indicating that the subject is in an upright orientation throughout the second time period with the upright orientation defined by the offset angle equaling or exceeding an offset angle threshold throughout the second time period, and as including subject change-in-position data indicating that a second rotation parameter of the subject change-in-position data is greater than a rotation threshold throughout the second time period. The motion monitoring system may further include an interface that is responsive to the processor, with the interface providing a first output from the processor in response to an identification of the first type of motion and providing a second output from the processor in response to an identification of the second type of motion.

In each of these embodiments, and in others, described below, the first type or seizure type of motion and the second type or the non-seizure type of motion may be characterized or further characterized by one or more of five motion parameters provided in the subject motion data, including the amplitude or magnitude of the detected motion, the period or frequency of the detected motion, the bandwidth of the detected motion, the position, orientation, or posture of the subject during the detected motion, and changes in the position, orientation, or posture of the subject during the detected motion. Those features may be expressed as values that include amplitude, period, bandwidth, offset angle, and rotation, and that may further include magnitude and frequency. Those values may be compared to ranges or thresholds to determine whether the detected motion is a first type of motion, a second type of motion, a seizure motion, or a non-seizure motion. Those ranges may include an amplitude range, a period range, a bandwidth range, an offset angle range or threshold, and a rotation range or threshold, and may further include a range or threshold expressed as a magnitude or a frequency.

The ranges and thresholds (for use in comparison to the detected motion) that are associated with the first type or the seizure type of motion may be preferred values that include: a first amplitude range that is at least one of 0.01 g to 0.60 g and 0.04 g to 0.48 g, a first period range that is at least one of 100 ms to 1000 ms and 160 ms to 750 ms, a first bandwidth range that is at least one of 0.05 to 0.60 and 0.10 to 0.50, an offset angle range that is at least one of zero degrees to 45 degrees and zero degrees to 60 degrees, and a rotation range that is at least one of zero degrees to 30 degrees and zero degrees to 20 degrees. The preferred ranges and thresholds associated with the first type or the seizure type of motion may be substituted by or used with alternative values that include: a first amplitude range that is at least one of 0.11 g to 0.50 g and 0.14 g to 0.38 g, a first period range that is at least one of 200 ms to 900 ms and 260 ms to 650 ms, a first bandwidth range that is at least one of 0.15 to 0.50 and 0.20 to 0.40, an offset angle range that is at least one of zero degrees to 35 degrees and zero degrees to 50 degrees, and a rotation range that is at least one of zero degrees to 20 degrees and zero degrees to 10 degrees.

The ranges and thresholds (for use in comparison to the detected motion) that are associated with the second type or the non-seizure type of motion may be preferred values that include: a second amplitude range that is at least one of 0.04 g to 1.00 g and 0.48 g to 1.00 g, a second period range that is at least one of 100 ms to 2000 ms and 100 ms to 1000 ms, a second bandwidth range that is at least one of zero to 0.80 and 0.10 to 0.80, an offset angle threshold that is at least one of 60 degrees and 45 degrees, and a rotation threshold that is at least one of 15 degrees and 30 degrees. The preferred ranges and thresholds associated with the second type or the non-seizure type of motion may be substituted by or used with alternative values that include: a second amplitude range that is at least one of 0.14 g to 0.90 g and 0.58 g to 0.90 g, a second period range that is at least one of 200 ms to 1900 ms and 200 ms to 900 ms, a second bandwidth range that is at least one of 0.10 to 0.70 and 0.20 to 0.70, an offset angle threshold that is at least one of 70 degrees and 55 degrees, and a rotation threshold that is at least one of 25 degrees and 40 degrees.

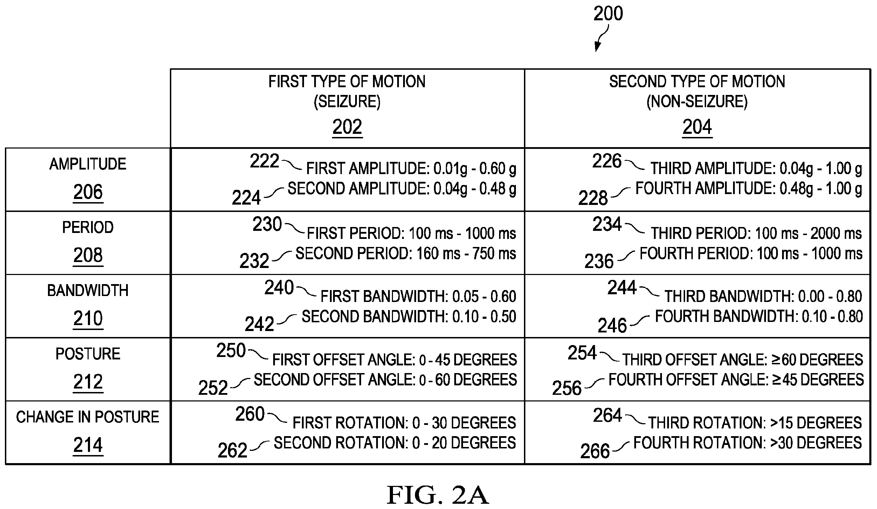

In each of these embodiments, and in others, described below, the detected motion may be compared to different sets of ranges and thresholds or combinations of ranges or thresholds to determine whether the motion is the first type or seizure type of motion or the second type or non-seizure type of motion. For example, subject motion data may be compared to the above-described ranges and thresholds relating to the preferred values for the first type of motion and compared to the above-described ranges and thresholds relating to the preferred values for the second type of motion to determine whether the detected motion is consistent with the first or second types of motion. In another example, subject motion data may be compared to the above-described ranges and thresholds relating to the alternative values for the first type of motion and compared to the above-described ranges and thresholds relating to the alternative values for the second type of motion to determine whether the detected motion is consistent with the first or second types of motion. In yet another example, subject motion data may be compared to the above-described ranges and thresholds relating to the preferred values for the first type of motion and compared to the above-described ranges and thresholds relating to the alternative values for the second type of motion to determine whether the detected motion is consistent with the first or second types of motion. In still another example, subject motion data may be compared to the above-described ranges and thresholds relating to the alternative values for the first type of motion and compared to the above-described ranges and thresholds relating to the preferred values for the second type of motion to determine whether the detected motion is consistent with the first or second types of motion. As further described below, the preferred and alternative ranges and threshold can each include different sets of ranges or threshold, thus providing two categories of preferred values and two categories of alternative values that can be selected when evaluating the detected motion. As can be appreciated, the ranges and thresholds provided in these categories can used in their entireties or used piecemeal with values of different categories being used to evaluate motion. For example, subject motion data may be compared to the above-described ranges and thresholds relating to a first category of the preferred values for the first type of motion (noted as "first" amplitude, period, etc. in FIG. 2A, for example) and compared to the above-described ranges and thresholds relating to the preferred values for the second type of motion (noted as "fourth" amplitude, period, etc. in FIG. 2A) to determine whether the detected motion is consistent with the first or second types of motion. In another example, subject motion data may be compared to the above-described ranges and thresholds relating to a modified first category of the preferred values for the first type of motion (using a mixed selection of "first" and "second" ranges for amplitude, period, etc. in FIG. 2A, for example) and compared to the above-described ranges and thresholds relating to the preferred values for the second type of motion (using a mixed selection of "third" and "fourth" ranges for amplitude, period, etc. in FIG. 2A) to determine whether the detected motion is consistent with the first or second types of motion.

In each of these embodiments, the comparison of the subject motion date to the selected ranges for the first/seizure type of motion and the second/non-seizure type of motion may be made to only a minimum or a maximum of the range instead of the entire range. The comparison may also be made over the entire duration of the motion time period, or for only a portion of the time period, or at only the beginning or end of the relevant time period, or using a combination of these comparison techniques. For example, the first amplitude may be compared to a minimum and/or a maximum of the first amplitude range, the first period may be compared to a minimum and/or a maximum of the first period range, the first bandwidth value may be compared to a minimum and/or a maximum of the first bandwidth range, the offset angle may be compared to a minimum and/or a maximum of the offset angle range, the first rotation parameter may be compared to a minimum and/or a maximum of the rotation range. Likewise, in another example, the second amplitude may be compared to a minimum and/or a maximum of the second amplitude range, the second period may be compared to a minimum and/or a maximum of the second period range, and the second bandwidth may be compared to a minimum and/or a maximum of the second bandwidth range.

For each of these embodiments, an identification of a first type or a seizure type of motion, and an identification of a second type or a non-seizure type of motion may be provided as an output or as a report to the subject or to a caregiver.

The features, functions, and advantages of the disclosed embodiments can be achieved independently in various embodiments or may be combined in yet other embodiments, further details of which are disclosed with reference to the following description and drawings.

### BRIEF DESCRIPTION OF THE DRAWINGS

FIG. 1A is a diagram of a particular illustrative embodiment of a sensor system defining a subject axis in a first orientation.
FIG. 1B is a diagram of a particular embodiment of the sensor system of FIG. 1A with the subject axis in a second orientation.
FIG. 2A is a table of illustrative preferred ranges and threshold values that may be used by a motion monitoring system to distinguish types of motion.
FIG. 2B is a table of illustrative alternative ranges and threshold values that may be used by a motion monitoring system to distinguish types of motion.
FIG. 3 is a diagram of a particular embodiment of a motion monitoring system to distinguish types of motion.
FIG. 4 is flow chart of a particular embodiment of a method that may be performed with a motion monitoring system to distinguish types of motion.
FIG. 5 is an illustration of a particular embodiment of a motion monitoring system illustrated in part in FIG. 1, including a sensor-patch component, a hub/programmer component, and a communication device component.
FIG. 6 is an illustration of exemplary placements of the sensor system of FIG. 1A on a subject.
FIGS. 7A-7B are further illustrations of the components of the motion monitoring system of FIG. 5.
FIGS. 8A-8B are further illustrations of the components of the motion monitoring system of FIG. 5.
FIGS. 9A-9C are further illustrations of the components of the motion monitoring system of FIG. 5.
FIG. 10 is a further illustration of the components of the motion monitoring system of FIG. 5.
FIG. 11 is an illustration of subject motion data obtained from a subject using the motion monitoring system of FIG. 5.

Illustrative embodiments are described herein. Particular illustrative embodiments of the present disclosure are described below with reference to the drawings. In the description, common elements are designated by common reference numbers throughout the drawings.

### DETAILED DESCRIPTION

A medical device system may include a motion monitoring system to gather and monitor motion data associated with a subject and perform seizure detection using the subject motion data. The monitoring or sensor system may generate a signal corresponding to the motion data and communicate the motion data to a base station system. The base station system may comprise a stand-alone communication device, a cellphone (mobile phone) device, or a combination thereof. The motion data may be sent by the base station system to a remote computing device associated with a healthcare provider, or a manufacturer or distributor of the medical device system, to monitor and perform additional medical diagnosis. The remote computing device may be associated with a care giver (e.g., a relative or an emergency care facility) of the user. The motion data may be sent by the base station system to the remote computing device to alert the care giver to a seizure event so that emergency medical services may be provided to the user. The motion data may be sent directly to a remote computing device to monitor, alert, or perform additional medical diagnoses. The motion data may also be processed at the sensor or at a portion of the sensor system containing or controlling the sensor, with the signal corresponding to the motion being transmitted to a base station or a remote computing device. Alternatively, the sensor or a portion of the sensor system containing or controlling the sensor may provide the data directly to the base station so as to perform most or all of the signal processing at the base station.

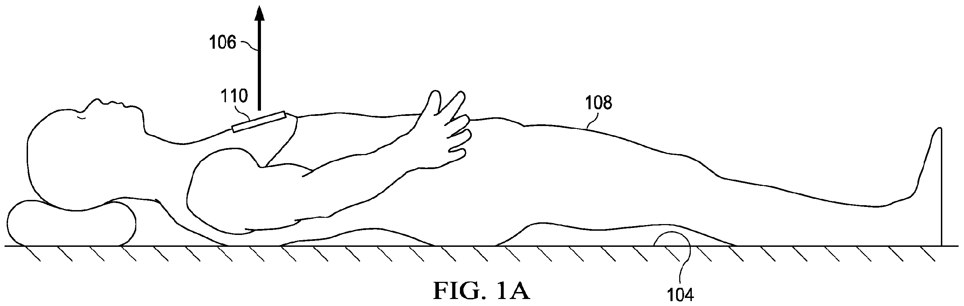

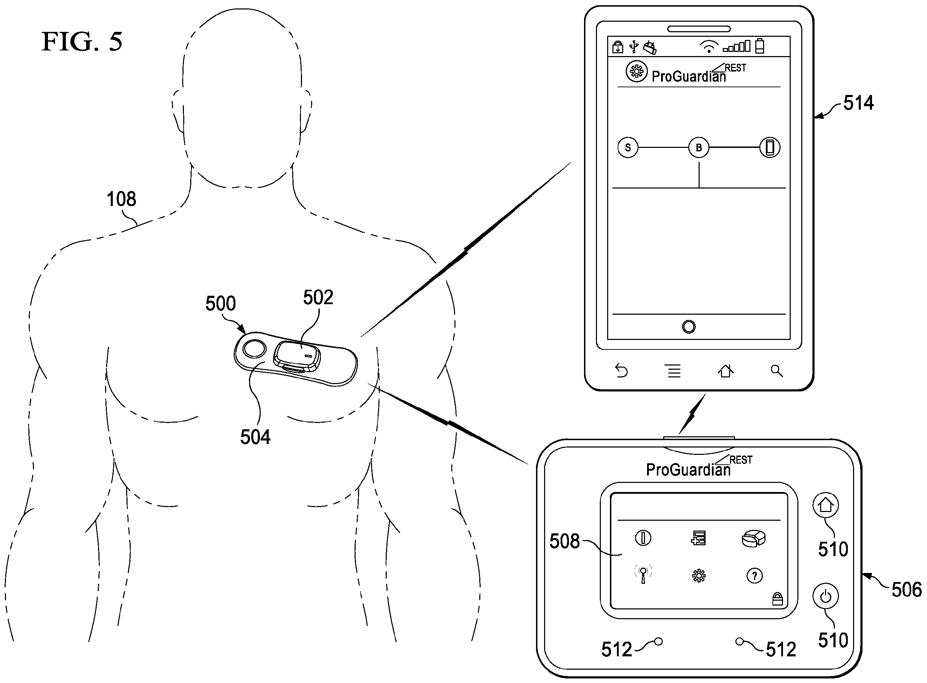

FIGS. 1A-1B illustrate diagrams of particular embodiments of a motion monitoring system or sensor system 110. FIGS. 5-10 provide further details regarding a particular embodiment of a sensor system 110. The sensor system 110 may be coupled to a subject (e.g., a user 108). For example, the sensor system 110 may be coupled to a patch and the patch may be coupled to the user 108. To illustrate, the patch may be placed on an exterior surface (e.g., skin) of the user 108. In a particular embodiment, the patch may be affixed (e.g., by an adhesive, a strap, or both) to the exterior surface (e.g., skin) of the user 108.

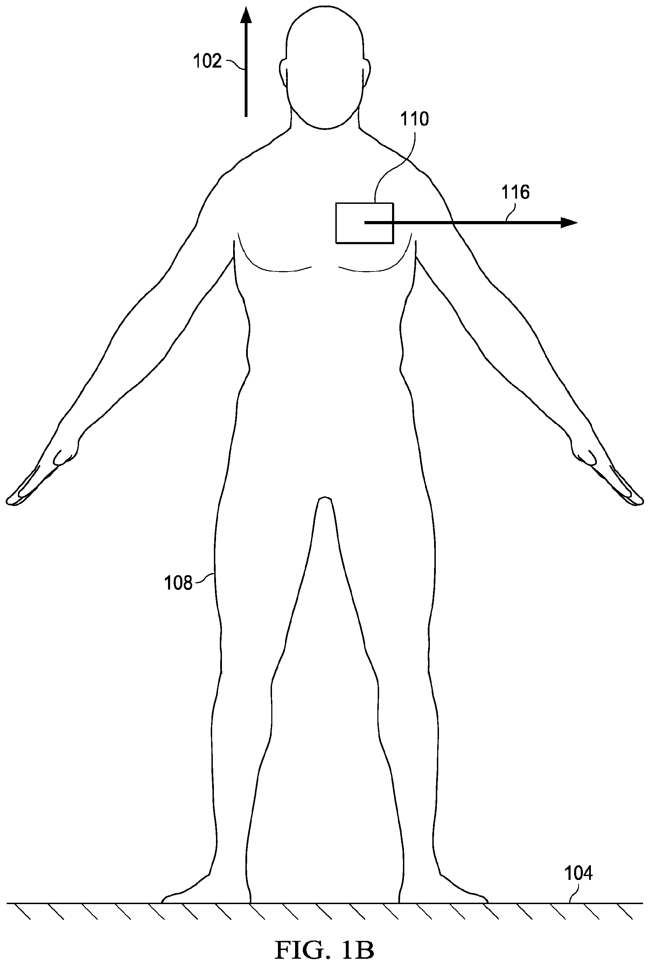

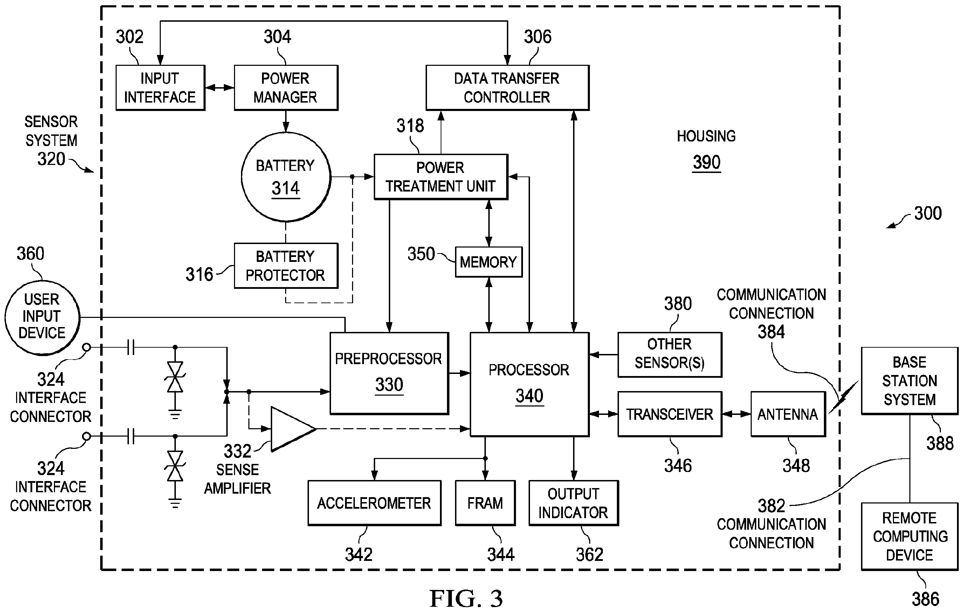

During operation, the sensor system 110 may be configured to detect and monitor movement of the user 108. For example, the sensor system 110 may include one or more accelerometers, as further described with reference to FIG. 3. The accelerometer may detect and monitor movement of the user 108. For example, the accelerometer may detect acceleration data corresponding to at least three axes (e.g., an x-axis, a y-axis, and a z-axis), and may detect the position of the subject and any change in the position of the subject. In another example, the sensor system 110 may have three accelerometers with each accelerometer disposed to detect acceleration data associated with a single axis (e.g., an x-axis, a y-axis, or a z-axis) and configured to generate the acceleration data as three distinct signals that can be collectively processed or combined at a later point or to generate the acceleration data as a single signal. The acceleration data may provide or be used to provide subject motion data, and that subject motion data may include data regarding the position of the subject (i.e., subject position data) and data regarding a change in the position of the subject (i.e., subject change-in-position data). The acceleration data, the subject motion data, the subject position data, and the subject change-in-position data may be based on data corresponding to any one or all of the three axes defined by the accelerometer or based on data corresponding to an angle relative to any of these axes, or a combination of position along an axis and an angle relative to an axis. The accelerometer may be mounted to a mounting system that includes a housing, and that is secured to an exterior surface of the user 108 with, for example, an adhesive layer. For example, the mounting system may be secured to a chest, a back, a shoulder, a side, or a limb of the user 108. As illustrated in FIGS. 1A and 1B, the sensor system 110 is secured to the chest of the subject 108.

A processor of the sensor system 110 may receive subject motion data associated with the subject 108 (e.g., from the accelerometer), or may receive accelerometer data from the accelerometer that includes subject motion data. The subject motion data or the acceleration data may include the subject position data and the subject change-in-position data. The subject motion data may be time sequenced and may relate to different periods of time. For example, the subject motion data may indicate acceleration data and timestamps associated with multiple measurement events. To illustrate, the accelerometer may detect the acceleration data periodically (e.g., at 10 millisecond intervals). The accelerometer data may be first and second accelerometer data and include, respectively, first subject motion data and first subject change-in-position data associated with a first timestamp (which may be expressed as a first time period) and second subject motion data and second change-in-position data associated with a second timestamp (which may be expressed as a second time period). The first timestamp may also indicate a first time period at which the first acceleration data is detected. The second timestamp may also indicate a second time period at which the second acceleration data is detected. As can be appreciated, the first acceleration data may correspond to a first type of motion by the subject that corresponds to a first period of time, and the second acceleration data may correspond to a second type of motion by the subject that corresponds to a second period of time. Furthermore, the first and second types of motions may be different types of motions and may concern different periods of time.

The sensor system 110 may be configured to detect a body position (e.g., posture) of the subject 108 relative to a vertical axis 102 defined based on gravitational pull, and the body position of the subject can be evaluated to determine whether the subject is in an recumbent orientation or an upright orientation. In one embodiment, the subject 108 can be disposed on a plane or surface 104 supporting the subject and the plane 104 can be at a 90 degree or similar angle relative to the vertical axis 102 to allow determination of the subject's orientation. The sensor system 110 may determine the position or orientation of the user 108 based on an angle formed by a subject axis (e.g., a subject axis in a first orientation 106 or a subject axis in a second orientation 116) relative to the vertical axis 102 or relative to the plane 104. The subject axis 106, 116 can extend from the subject 108 or the sensor system 110 in a normal direction away from the subject 108 or sensor system 110 to provide a position or orientation of the subject 108 or sensor system 110 relative to a frame of reference defined by the vertical axis 102 or the plane 104. As can be appreciated, the subject axis 106, 116 may not be perpendicular or parallel to the vertical axis 102 or the plane 104 and instead be offset by an angle that can be identified and accounted for when determining subject position or orientation relative to an external reference such as vertical axis 102 or plane 104. As can also be appreciated, the position or orientation of the subject axis 106, 116 and the offset or offset angle used to relate the subject axis 106, 116 to an external frame of reference may be based on an initial calibration of the sensor system 110 or the accelerometer or accelerometers supported by the sensor system 110 that is made when the subject 108 is in a known position or orientation to the vertical axis 102 or to the plane or surface 104. The calibration may provide a baseline orientation of the subject from which the offset or offset angle can be defined. The subject axis 106, 116 may be an axis extending from the subject 108 or the sensor system 110 that is compared to the vertical axis 102 or plane 104 to identify an offset or offset angle between the subject axis 106, 116 and the vertical axis 102 and to identify whether the subject is in a recumbent position or an upright position. The angle between the subject axis 106, 116 and the vertical axis 102 or the plane 104 may define an initial position of the subject axis 106, 116 relative to the vertical axis 102 or plane 104 and subsequent positions of the subject axis 106, 116 may be identified and compared to the vertical axis 102 or plane 104. Likewise, a similar comparison can be made to identify an initial position of the subject axis 106, 116 to determine any change in the subject's position over time. As can be appreciated, such change-in-position data can be compared to the first or second timestamps or to the first or second time periods to determine a rate at which the subject changes position.

The subject axis (e.g., the subject axis in the first orientation 106 or the subject axis in the second orientation 116) may extend away from the user 108 in a direction normal to a frontal plane of the user 108, which may be understood to be a coronal plane of the subject's body and may be further understood to be a plane that divides the subject's body into ventral and dorsal sections. The coronal plane of the subject can be parallel or nearly parallel to the plane 104 when the subject in the recumbent orientation, and the coronal plane can extend in a direction that is parallel or nearly parallel to the vertical axis 102 when the subject is in the upright position. In a particular embodiment, the accelerometer may be coupled to the subject (e.g., the user 108) to define the subject axis. For example, the accelerometer may be coupled to a chest of the user 108 and the subject axis may extend away from the user 108 or the sensor system 110 in a direction perpendicular (normal) to the subject's chest. As another example, the accelerometer may be coupled to a back of the user 108 and the subject axis may extend away from the user in a direction perpendicular to the subject's back. In still another example, the accelerometer may be coupled to any portion of the subject to provide a subject axis extending away from the subject, and the subject axis established by the accelerometer may be calibrated with the vertical axis 102 or with some other reference point. The calibration may provide a baseline orientation of the subject from which the offset or offset angle can be defined. The accelerometer may detect acceleration about multiple axes to determine a relative orientation of the subject axis with respect to the vertical axis 102.

As illustrated in FIG. 1A, in a first orientation, the user 108 may be lying down on the surface 104 in a recumbent orientation with the sensor system 110 disposed on the chest of the subject. The subject axis in the first orientation 106 may extend away from the user 108 in an upward direction normal to a frontal plane of the subject 108. Thus, in the first orientation 106, the accelerometer may detect a first angle (e.g., substantially 0 degrees) between the subject axis in the first orientation 106 and the vertical axis 102, and also define an initial first position of the subject axis relative to the vertical axis 102. As can be appreciated, the first angle may be greater than zero and may correspond to an offset or an offset angle that may be used to account for any misalignment between the subject axis and an external frame of reference such as the vertical axis 102. As illustrated in FIG. 1B, in a second orientation, the subject 108 may be standing on the surface 104 or sitting with the subject's chest in an upright orientation. The subject axis in the second orientation 116 may extend away from the user 108 in a horizontal direction normal to a frontal plane of the subject 108. Thus, in the second orientation, the accelerometer may detect a second angle (e.g., substantially 90 degrees) between the subject axis in the second orientation 116 and the vertical axis 102, and also define an initial second position of the subject axis relative to the vertical axis 102. As explained with regard to the recumbent orientation, the second angle may be greater or less than 90 degrees and may correspond to an offset or an offset angle that may be used to account for any misalignment between the subject axis and an external frame of reference such as the vertical axis 102. The subject motion data provided by the accelerometer to the processor may identify the posture of the subject 108 in terms of the angle (e.g., 0 degrees or 90 degrees) formed by the subject axis relative to the vertical axis 102, as modified when considering the offset angle or the initial calibration of the device used to determine the subject's position and any change in the subject's position. Thus, the posture may indicate or correspond to a position of the subject 108. As can be appreciated from the embodiment of FIGS. 1A and 1B, an angle of 0 degrees or an angle of less than 45 degrees could possibly indicate that the subject is laying down (e.g., in a recumbent orientation) while an angle of more than 45 degrees or an angle 90 degrees could possible indicate that the subject is standing or sitting up (e.g., in an upright orientation). As can also be appreciated, the subject can be at inclined orientations that are translatable to the recumbent and upright orientations, or translatable to the normal direction of the subject axis, so as to account for variations in the positioning and coupling of the sensor system 110 to the subject's skin.

The sensor system 110 may be configured to detect subject position (or posture) and subject change-in-position (or change of posture) over a time period or time window. The time period may be any suitable length of time extending between an initial determination of the subject position and a subsequent determination of the subject position and, as an example, can be 1 ms, 10 ms, 100 ms, 1 second, 10 seconds, 1 minute, or any increment between these values. The time period can be established to best suit the type of motion detected, the quality of the sensed motion data, the type of seizure to be detected, subject body type factors, the location of the motion monitoring system relative to the body, and the power levels and battery capacity of the system. As can be appreciated, a first time period may be associated with a first type of motion and a second time period may be associated with a second type of motion that may be a different type of motion than the first type of motion. As can be further appreciated, the time period can be constant for the first type of motion and the second type of motion, or the time window for the first type of motion can be different than the time window for the second type of motion. As can be further appreciated, the time periods for the first and second types of motion can be determined independently, be based on different parameters or values, share common parameters, or be constructed so that the time period for one type of motion is a function of the time period for another type of motion. The initial and subsequent determinations of subject position can each be relative to the vertical axis 102 or, alternatively, relative to each other. The initial and subsequent subject positions can define a varying angle between the initial and subsequent positions of the subject axis. The varying angle can have a value in degrees that the subject axis has moved over a period of time defined by the time period. For example, the posture of the user 108 may be changing from a recumbent or lying down orientation (e.g., as illustrated in FIG. 1A) to an upright or standing up orientation (e.g., as illustrated in FIG. 1B). The sensor system 110 may detect the change in posture based on a change associated with the position of the subject axis (e.g., as a number of degrees) over the selected time period. For example, the subject motion data may indicate that at a first time (e.g., 9:00:00.000 AM) the subject axis was parallel (e.g., 0 degrees) to the vertical axis 102 and may indicate that at a second time (e.g., 9:00:03.000 AM) the subject axis was perpendicular (e.g., 90 degrees) to the vertical axis 102. The processor may determine the change in posture based on a difference between the angle of subject axis and the vertical axis 102 (e.g., 90 degrees - 0 degrees) and then determine that an appropriate time period is the difference (e.g., 3 seconds) between the first time and the second time. In a variation of this example, the time period may be determined to be the 1-second period disposed in the middle of the measured 3-second period because of unreliability detected in the data obtained during the initial and last seconds of the 3-second period measured in this example. In another variation of this example, the time period may be determined to be 100 ms of the 3-second measurement period because the other parameters measured by the system (e.g., amplitude, period, bandwidth) were determined to be most reliable during the selected 100 ms portion of the 3-second measurement period.

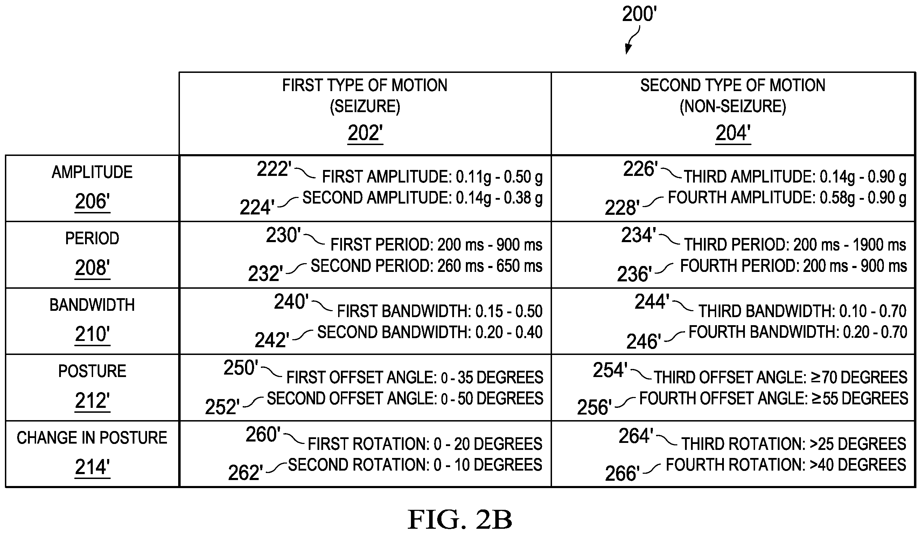

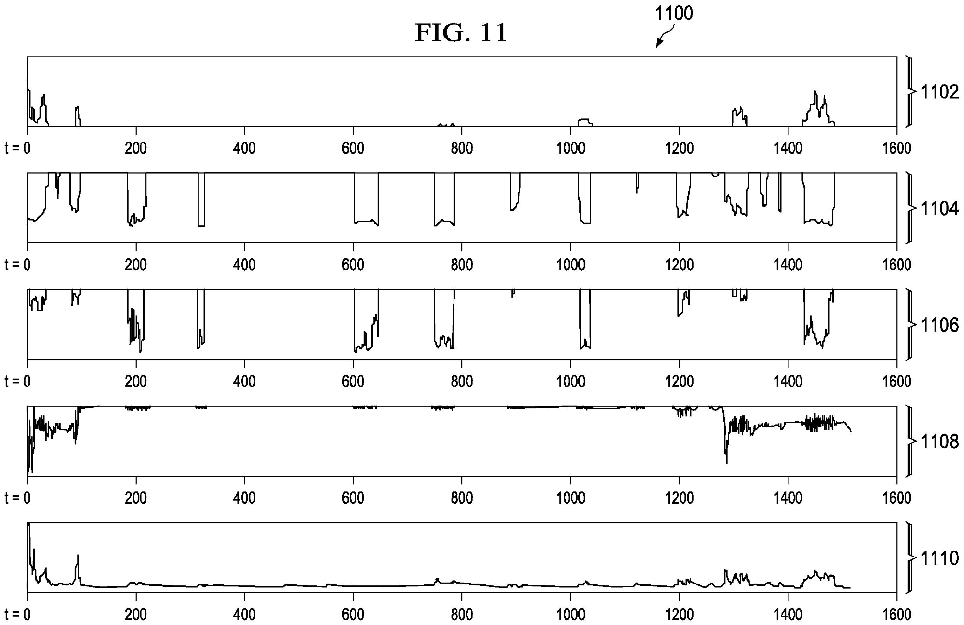

The sensor system 110 may be configured to detect an amplitude or magnitude 206 associated with the movement of the subject 108 and may be configured to generate a signal corresponding to the detected motion of the subject, such as the amplitude signal 1102 illustrated in FIG. 11. A motion sensor, such as an accelerometer, may generate the signal, and that signal may include subject motion data that may be expressed as an amplitude or magnitude value, which is a representation of the intensity of the subject's motion. In an embodiment, the accelerometer (or another sensor, such as a camera) may periodically (e.g., at 3 millisecond intervals) detect gravitational force or acceleration in a three-dimensional (x, y, and z) domain and generate a signal that includes an amplitude component. The amplitude value provided by the motion sensor may be expressed as a root mean square (RMS) amplitude, and the amplitude value may be an envelope of the motion sensor signal that is proportional to the RMS amplitude. The amplitude value derived from the motion sensor signal may be expressed as a single value representing the amplitude for a time period associated with the detected motion, may be expressed as a value representative of the entire time period associated with the detected motion, and may be expressed as value representative of discrete points of the associated time period such as at the beginning or end of the time period. The amplitude value may also be expressed as an average over the time period, or expressed as a value representing the extremes or peaks of the signal over the time period. The amplitude value may also be provided as a first amplitude associated with a first type of motion occurring over a first time period, and as a second amplitude associated with a second type of motion occurring over a second time period. The amplitude value may also be in a form that allows comparison to a range or a subset of a range such as the preferred ranges provided in FIG. 2A, or the alternative ranges provided in FIG. 2B, at first amplitude range 222 (or 222'), second amplitude range 224 (or 224'), third amplitude range 226 (or 226'), and fourth amplitude range 228 (or 228'). For example, a first amplitude value can be compared to a first amplitude range to determine whether the first amplitude value falls within the first amplitude range, and the same comparison may be made for the second amplitude value to a second amplitude range. As can be appreciated, the comparison may evaluate where the detected amplitude value stands with respect to the amplitude range for the entire length of the corresponding time period, and require that the detected amplitude value remains within the amplitude range for the entire time period. Alternatively, the same comparison may be made only at a portion of the corresponding time period, with the evaluating looking at only whether the amplitude value is within or outside of the amplitude range at certain points within the corresponding time period, such as at the beginning or end of the time period. In another alternative, the same comparison may be made with the evaluating looking at only whether the detected amplitude value exceeds the amplitude range or an amplitude threshold representative of the amplitude range, with the evaluation focused on instances where the amplitude value falls outside of the amplitude range at any point during the corresponding time period, or at portions of the corresponding time period such as at a beginning or an end of the time period or the beginning or the end of a portion of the time period. The same comparison may be made between the detected amplitude value and a minimum and/or a maximum of the amplitude range. The amplitude value can be expressed in units of "g" representing acceleration.

The sensor system 110 may be configured to detect a period or frequency 208 associated with the movement of the subject 108 and may be configured to generate a signal corresponding to the detected motion of the subject, such as the period signal 1104 illustrated in FIG. 11. As can be appreciated, the frequency detected by the sensor system 110 may be used to provide a value for a period corresponding to at least a portion of the signal, and the period detected by the sensor system 110 may be used to provide a value for a frequency associated with the signal. The motion sensor, such as an accelerometer, may generate the signal, and that signal may include subject motion data that may be expressed as a period or frequency value, which is a representation of the rhythmicity of the subject's motion. In an embodiment, the subject motion data associated with one or more axes of the three-dimensional (3D) domain may be periodic (e.g., have a time varying amplitude). The processor may determine the period by calculating a time difference (e.g., 100 ms) between a first amplitude peak and a second amplitude peak or by calculating a time difference between zero crossings. As can be appreciated, the processor may also determine the frequency value using known methods and may base the determination of frequency on the period value. The period or frequency value provided by the motion sensor may be expressed as a function of time and may correspond to the time period associated with the detected motion. The period or frequency value derived from the motion sensor signal may be expressed as a single value representing the period or frequency for a time period associated with the detected motion, may be expressed as a value representative of the entire time period associated with the detected motion, and may be expressed as value representative of discrete points of the associated time period such as at the beginning or end of the time period. The period or frequency value may also be expressed as an average over the time period, or expressed as a value representing the extremes of the signal over the time period. The period or frequency value may also be provided as a first period or frequency associated with a first type of motion occurring over a first time period, and as a second period or frequency associated with a second type of motion occurring over a second time period. The period or frequency value may also be in a form that allows comparison to a range or a subset of a range such as the preferred ranges provided in FIG. 2A, or the alternative ranges provided in FIG. 2B, at first period range 230 (or 230'), second period range 232 (or 232'), third period range 234 (or 234'), and fourth period range 236 (or 236'). For example, a first period or frequency value can be compared to a first period or frequency range to determine whether the first period or frequency value falls within the first period or frequency range, and the same comparison may be made for the second period or frequency value to a second period or frequency range. As can be appreciated, the comparison may evaluate where the detected period or frequency value stands with respect to the period or frequency range for the entire length of the corresponding time period, and require that the detected period or frequency value remains within the period or frequency range for the entire time period. Alternatively, the same comparison may be made only at a portion of the corresponding time period, with the evaluating looking at only whether the period or frequency value is within or outside of the period or frequency range at certain points within the corresponding time period, such as at the beginning or end of the time period. In another alternative, the same comparison may be made with the evaluating looking at only whether the detected period or frequency value exceeds the period or frequency range or an period or frequency threshold representative of the period or frequency range, with the evaluation focused on instances where the period or frequency value falls outside of the period or frequency range at any point during the corresponding time period, or at portions of the corresponding time period such as at a beginning or an end of the time period or the beginning or the end of a portion of the time period. The same comparison may be made between the detected period or frequency value and a minimum and/or a maximum of the period or frequency range. The period value may be expressed in units of time such as milliseconds (ms) representing the duration of one cycle of a repeating event in the signal. The frequency value may be expressed as a number of occurrences if a repeating event in the signal per unit of time in units of hertz (Hz).

The sensor system 110 may be configured to detect a bandwidth 210 associated with the movement of the subject 108 and may be configured to generate a signal corresponding to the detected motion of the subject, such as the bandwidth signal 1106 illustrated in FIG. 11. The motion sensor, such as an accelerometer, may generate the signal, and that signal may include subject motion data that may be expressed as a bandwidth value, which is a representation of the coordination of the subject's motion and/or a representation of the variability in the period of the movement of the subject 108. In an embodiment, a processor may determine an average of several periods indicated by the subject motion data (e.g., a time windowed moving average period). The processor may also calculate the bandwidth value associated with a particular period by determining a ratio of the particular period and the average period. The bandwidth value provided by the motion sensor may be expressed as a function of time and may correspond to the time period associated with the detected motion. The bandwidth value derived from the motion sensor signal may be expressed as a single value representing the bandwidth for a time period associated with the detected motion, may be expressed as a value representative of the entire time period associated with the detected motion, and may be expressed as value representative of discrete points of the associated time period such as at the beginning or end of the time period. The bandwidth value may also be expressed as an average over the time period, or expressed as a value representing the extremes of the signal over the time period. The bandwidth value may also be provided as a first bandwidth associated with a first type of motion occurring over a first time period, and as a second bandwidth associated with a second type of motion occurring over a second time period. The bandwidth value may also be in a form that allows comparison to a range or a subset of a range such as the preferred ranges provided in FIG. 2A, or the alternative ranges provided in FIG. 2B, at first bandwidth range 240 (or 240'), second bandwidth range 242 (or 242'), third bandwidth range 244 (or 244'), and fourth bandwidth range 246 (or 246'). For example, a first bandwidth value can be compared to a first bandwidth range to determine whether the first bandwidth value falls within the first bandwidth range, and the same comparison may be made for the second bandwidth value to a second bandwidth range. As can be appreciated, the comparison may evaluate where the detected bandwidth value stands with respect to the bandwidth range for the entire length of the corresponding time period, and require that the detected bandwidth value remains within the bandwidth range for the entire time period. Alternatively, the same comparison may be made only at a portion of the corresponding time period, with the evaluating looking at only whether the bandwidth value is within or outside of the bandwidth range at certain points within the corresponding time period, such as at the beginning or end of the time period. In another alternative, the same comparison may be made with the evaluating looking at only whether the detected bandwidth value exceeds the bandwidth range or a bandwidth threshold representative of the bandwidth range, with the evaluation focused on instances where the bandwidth value falls outside of the bandwidth range at any point during the corresponding time period, or at portions of the corresponding time period such as at a beginning or an end of the time period or the beginning or the end of a portion of the time period. The same comparison may be made between the detected bandwidth value and a minimum and/or a maximum of the bandwidth range. The bandwidth may be expressed as a the difference between the upper and lower frequencies in a contiguous set of frequencies associated with the detected motion over the relevant time period, and presented as a unit-less number or in units of hertz (Hz).

The sensor system 110 may be configured to detect a position or orientation (or posture) 212 of the subject 108 and may be configured to generate a signal corresponding to the detected position or orientation of the subject, such as the position signal 1108 illustrated in FIG. 11. A motion sensor, such as an accelerometer, may generate the signal, and that signal may include subject motion data that may be expressed as a position or orientation value, which is a representation of the posture of the subject. The position or orientation value may be expressed as an offset angle representing a difference between a subject axis 106 extending from the subject relative to an external frame of reference defined by, for example, vertical axis 102. As described above, in an embodiment the accelerometer (or another sensor, such as a camera) may be coupled to the subject 108 or configured to identify a position of the subject relative to the external frame of reference, such as a vertical axis 102 or a plane 104, and with calibration and/or the use of an offset, if necessary, the subject's position or orientation can be determined from a variety of configurations in which the calibration or offset establishes a relationship between the detected position or orientation value and the actual position or orientation of the subject. The position or orientation value may indicate that the subject is in a recumbent position, an upright position, a partially upright position, an unclassified position, or a combination of these positions. The position or orientation value may also indicate whether the subject 108 is reclining, sitting, standing, or laying down on the subject's back, front, or side. The position or orientation value derived from the motion sensor signal may be expressed as a single value representing the position or orientation for a time period associated with the detected motion, may be expressed as a value representative of the entire time period associated with the detected motion, and may be expressed as value representative of discrete points of the associated time period such as at the beginning or end of the time period. The position or orientation value may also be expressed as an average over the time period, or expressed as a value representing the extremes or peaks of the signal over the time period. The position or orientation value may also be provided as an offset angle value that may be associated with a first type of motion occurring over a first time period and/or associated with a second type of motion occurring over a second time period. The position or orientation value may also be in a form that allows comparison to a range or a subset of a range or to a threshold such as the preferred ranges and thresholds provided in FIG. 2A, or the alternative ranges and thresholds provided in FIG. 2B, at first angle range 250 (or 250'), second angle range 252 (or 252'), third angle threshold 254 (or 254'), and fourth angle threshold 256 (or 256'). For example, an offset angle value associated with the first time period can be compared to an offset angle range to determine whether the offset angle value falls within the offset angle range, and a similar comparison may be made for an offset angle value associated with the second time period to determine whether the offset angle range value is greater or less than a offset angle threshold. As can be appreciated, the use of ranges and thresholds in this embodiment can be switched to account for different configurations of the subject relative to the external frame of reference, or to account for use of different frames of reference. As can be further appreciated, the comparison may evaluate where the detected offset angle value stands with respect to the offset angle range or offset angle threshold for the entire length of the corresponding time period, and require that the detected offset angle value remains within the offset angle range or below the offset angle threshold for the entire time period. Alternatively, the same comparison may be made only at a portion of the corresponding time period, with the evaluating looking at only whether the offset angle value is within or outside of the offset angle range or offset angle threshold at certain points within the corresponding time period, such as at the beginning or end of the time period. In another alternative, the same comparison may be made with the evaluating looking at only whether the detected offset angle value exceeds the offset angle range or offset angle threshold, with the evaluation focused on instances where the offset angle value falls outside of the offset angle range or exceeds the offset angle threshold at any point during the corresponding time period, or at portions of the corresponding time period such as at a beginning or an end of the time period or the beginning or the end of a portion of the time period. The same comparison may be made between the detected offset angle value and a minimum and/or a maximum of the offset angle range. The offset angle value, offset angle range, and offset angle threshold may be expressed in units of degrees.

The sensor system 110 may be configured to detect a change in position or a change in orientation (or change in posture) 214 of the subject 108 and may be configured to generate a signal corresponding to the detected change in position or change in orientation of the subject, such as the change-in-position signal 1110 illustrated in FIG. 11. A motion sensor, such as an accelerometer, may generate the signal, and that signal may include subject motion data that may be expressed as a change-in-position or change-in-orientation value, which is a representation of a change in the posture of the subject. The change-in-position or change-in-orientation value may be expressed as a rotation parameter representing a rotation of the subject over the time period corresponding to the detected motion. As described above, in an embodiment the accelerometer (or another sensor, such as a camera) may be coupled to the subject 108 or configured to identify an initial position of the subject at a beginning of the time period and a later position of the subject farther along in the time period or at the end of the time period so as to determine the magnitude and direction of the rotation of the subject and, with the time period value, determine a rate of the rotation of the subject. The change-in-position or change-in-orientation value may indicate that the subject is rotating in a way that is or is not seizure related, rotating in a way that is associated with a normal motion of a non-seizure activity, rotating in an unclassified manner, or a combination of these rotations. The change-in-position or change-in-orientation value may also indicate whether the subject 108 is rolling in a sleep state, rhythmically walking, jumping, or moving in a way consistent with a normal non-seizure activity. The change-in-position or change-in-orientation value derived from the motion sensor signal may be expressed as a single value representing the rotation of the subject for a time period associated with the detected motion, may be expressed as a value representative of the entire time period associated with the detected motion, and may be expressed as value representative of discrete points of the associated time period such as at the beginning or end of the time period. The change-in-position or change-in-orientation value may also be expressed as an average over the time period, or expressed as a value representing the extremes or peaks of the signal over the time period. The change-in-position or change-in-orientation value may also be provided as a first rotation value or parameter that may be associated with a first type of motion occurring over a first time period, and may be provided as a second rotation value or parameter that may be associated with a second type of motion occurring over a second time period. The change-in-position or change-in-orientation value may also be in a form that allows comparison to a rotation range or a subset of a rotation range or to a rotation threshold such as the preferred ranges and thresholds provided in FIG. 2A, or the alternative ranges and thresholds provided in FIG. 2B, at first rotation/rate range 260 (or 260'), second rotation/rate range 262 (or 262'), third rotation threshold/rate threshold 264 (or 264'), and fourth rotation threshold/rate 266 (266'). For example, a first rotation value or parameter associated with the first time period can be compared to an rotation range to determine whether the first rotation value falls within the rotation range, and a similar comparison may be made for a second rotation value or parameter associated with the second time period to determine whether the second rotation value is greater or less than a rotation threshold. As can be appreciated, the use of ranges and thresholds in this embodiment can be switched to account for different configurations of the subject relative to the external frame of reference, or to account for use of different frames of reference. As can be further appreciated, the comparison may evaluate where the detected rotation value stands with respect to the rotation range or rotation threshold for the entire length of the corresponding time period, and require that the detected rotation value remains within the rotation range or below the rotation threshold for the entire time period. Alternatively, the same comparison may be made only at a portion of the corresponding time period, with the evaluating looking at only whether the rotation value is within or outside of the rotation range or rotation threshold at certain points within the corresponding time period, such as at the beginning or end of the time period. In another alternative, the same comparison may be made with the evaluating looking at only whether the detected rotation value exceeds the rotation range or rotation threshold, with the evaluation focused on instances where the rotation value falls outside of the rotation range or exceeds the rotation threshold at any point during the corresponding time period, or at portions of the corresponding time period such as at a beginning or an end of the time period or the beginning or the end of a portion of the time period. The same comparison may be made between the detected rotation value and a minimum and/or a maximum of the rotation range. The rotation value, rotation range, and rotation threshold may be expressed in units of degrees.

In a particular embodiment, the processor may analyze the posture, the change of the posture, the amplitude, the period, the bandwidth, or a combination thereof, associated with a particular axis (e.g., the x-axis, the y-axis, and the z-axis) of the 3D domain. For example, the processor may determine an axis amplitude associated with each of the three axes. The processor may determine the axis amplitude based on a high peak and a low peak associated with each axis (e.g., the x-axis, the y-axis, or the z-axis) during a sample time window. As another example, the processor may determine an axis posture based on an axis orientation (e.g., an x-axis orientation, a y-axis orientation, or a z-axis orientation) relative to the vertical axis 102. As a further example, the processor may determine an axis change in posture based on a change of the axis orientation. As another example, the processor may determine an axis period based on a time difference associated with peaks or zero crossing corresponding to a particular axis (e.g., the x-axis, the y-axis, or the z-axis). As a further example, the processor may determine an axis bandwidth based on a ratio of a particular axis period and an average axis period.

In a particular embodiment, the processor may use axis measurements associated with a particular axis to distinguish between various types of motion (e.g., seizure motions and non-seizure motions). For example, when the user 108 is sleeping, one or more of the axis measurements (e.g., posture, change of posture, amplitude, period, or bandwidth) associated with the y-axis may have higher values than the axis measurements associated with the x-axis and the z-axis. The processor may use the axis measurements associated with the y-axis in distinguishing between the various types of motions, as further described with reference to FIG. 2A. In another embodiment, the processor may use the axis measurements that indicate a common type of motion to distinguish between the various types of motion. For example, the processor may choose the type of motion indicated by a highest number of axis measurements. To illustrate, the x-axis measurements and the y-axis measurements may indicate a first type of motion and the z-axis measurements may indicate a second type of motion. The processor may indicate the first type of motion because more (e.g., 2 out of 3) of the axis measurements indicate the first type of motion. In another embodiment, the processor may use the axis measurement that most clearly distinguishes between the various types of motion. For example, the x-axis measurements may indicate a first type of motion, the y-axis measurements may indicate a second type of motion, and the z-axis measurements may be inconclusive. The processor may use the x-axis measurements in response to determining that more of the x-axis measurements (e.g., amplitude, period, posture, change of posture, and/or bandwidth) correspond to the first type of motion than the y-axis measurements corresponding to the second type of motion. In a particular embodiment, the processor may use the x-axis measurements in response to determining that the x-axis measurements are well within ranges of threshold values corresponding to the first type of motion, whereas the y-axis measurements are nearer the limits of ranges of threshold values corresponding to the second type of motion.

The motion of a subject can be monitored and measured in a variety of ways and with a variety of measurement devices. Preferably, the motion of a subject can be observed by a motion monitoring device or system oriented to receive data from the subject as the subject moves. The motion monitoring device can be configured to collect a single parameter of motion, such as velocity, or collect multiple parameters of motion, such as velocity and direction. The motion monitoring device can be configured to collect motion data that is combined with motion data obtained from another device, which can be another motion monitoring device or system, or a device or system that does not measure motion directly, such as a pressure sensor. The monitoring can be indirect, such as with a video or visual system that can record the motion of a subject from a distance. One example of an indirect motion monitoring device is a Kinect motion monitoring system provided with some Microsoft gaming systems, which remotely monitors the motion of persons operating the system. Other examples on indirect motion monitoring systems include a video camera and an RF motion detector. In still another example of an indirect motion monitoring system, the aforementioned monitoring systems can be mounted on the subject, e.g., as a camera on a helmet, and the indirect motion monitoring is derived from how the subject-mounted system moves relative to a stationary environment viewed by the monitoring system. The monitoring can also be direct, with the motion of the subject measured by the placement of a sensor on or in a defined relationship to the subject's body. Preferably, the direct motion monitoring system is an accelerometer affixed to a subject body, preferably to a portion of the subject's body that is adjacent to the subject's skeleton. More preferably, the direct motion monitoring system is an accelerometer affixed to the surface of the subject's skin at the subject's chest, placed over the rib cage or over the sternum. The direct motion monitoring system may be coupled to the subject's limbs, such as the ankle or wrist. The direct motion monitoring system may be affixed to the subject with an adhesive or held fast with tape or a strap, or the system may be embedded in another structure such as on or within an item of clothing, jewelry, or a watch. The direct motion monitoring system may also have components that are implanted within a subject.

A motion monitoring system, such as an accelerometer, may monitor and provide motion data corresponding to activity and movement associated with a patient's body. The motion data may be used as an alternative to, or in addition to, other data (such as ECG data or EEG data) to identify a seizure. The motion monitoring system may be attached to an external surface of the patient's body. The motion data may be associated with movement of the patient's chest or with a change or a rate of change of a patient's position (such as associated with the patient moving from a lying position to a sitting position). The motion data may be compared with threshold values to distinguish a first type of motion (e.g., seizure motion) from a second type of motion (e.g., non-seizure motion). For example, the first type of motion may be associated with first threshold values of posture, amplitude, period, bandwidth, subject position or axis, and/or change or rate of change of the subject position or axis. As another example, the second type of motion may be associated with second threshold values of posture, amplitude, period, bandwidth, subject position or axis, and/or change or rate of change of the subject axis. The motion data may be analyzed to distinguish between the first type of motion and the second type of motion by determining whether the motion data satisfies one or more of the first threshold values or one or more of the second threshold values.

In a particular embodiment, the sensor system 110 may use one or more other sensors alternatively, or in addition to, the accelerometer to detect the subject motion data. The one or more other sensors may be coupled to a chest, a back, a shoulder, a side, or a limb of the user 108. One example of an additional sensor is a gyroscope that may be used to detect the subject motion data and, in particular, to detect the rotation of the subject.

In a particular embodiment, the one or more other sensors may gather data regarding the user 108 at a distance from the user 108. For example, the sensor system 110 may include or be coupled to a visualization device that may include a video device such as a camera or a thermal imaging system and/or may include or be coupled to a motion detector, a depth sensor, or an infrared laser device. As can be appreciated, in some embodiments the sensor system 110 may provide a two dimensional image with subject motion data obtained via the video device and may provide data regarding a third dimension with the motion detector, depth sensor, or infrared laser device. The sensor system 110 may be located in a same room as the user 108. The sensor system 110 may periodically capture images of the user 108. The subject motion data may be generated by sensor system 110 based on the images obtained by a video device alone or in association with other devices such as the motion detector, depth sensor, or infrared laser device. For example, the sensor system 110 may identify a frontal plane of the user 108 presented in a first image of the user 108 and may define the subject axis in the first orientation 106 extending in a direction normal to the frontal plane. As another example, the sensor system 110 may determine a position of the user 108 in the at least 3-axes (e.g., the x-axis, the y-axis, and the z-axis) based on an analysis of the images obtained by the sensor system 110 or the camera of the sensor system 110. The sensor system 110 may also determine a posture, a change of the posture, an amplitude, a period and a bandwidth associated with movements of the user 108 in the at least 3-axes (e.g., the x-axis, the y-axis, and the z-axis) based on the analysis of the images obtained with the sensor system 110.

The sensor system 110 may be configured to distinguish between various types of motion by analyzing the subject motion data, as further described with reference to FIG. 2A. For example, the sensor system 110 may distinguish between seizure motion and non-seizure motion by comparing the posture, the change of the posture, the amplitude, the period, the bandwidth, or a combination thereof to threshold values or ranges. The processor may generate an output in response to identification of a particular type of motion (e.g., seizure motion, non-seizure motion, or both). For example, the sensor system 110 may transmit the subject motion data, the output, or both, via a transmitter, to a base station system.

Referring to FIG. 2A, a table of illustrative preferred ranges and threshold values are disclosed and generally designated 200. FIG. 2B provides an alternative table 200' of illustrative ranges and threshold values that can be substituted for or used with the values for FIG. 2A. One or more of the threshold values indicated by the table 200 (or table 200') may be stored at, or be accessible to, the sensor system 110 of FIG. 1. For example, the sensor system 110 may receive the one or more threshold values via user input. As another example, the one or more threshold values may correspond to default values.

The table 200 (or 200') includes a first column associated with a first type of motion 202/202' (e.g., seizure motion) and a second column associated with a second type of motion 204/204' (e.g., non-seizure motion). The table 200 (200') also includes a first row associated with amplitude range values 206 (206'), a second row associated with period range values 208 (208'), a third row associated with bandwidth range values 210 (210'), a fourth row associated with posture range and threshold values 212 (212'), and a fifth row associated with change of posture range and threshold values 214 (214'). The table 200 indicates ranges and threshold values that are indicative of the first type of motion 202 and of the second type of motion 204.

During operation, the sensor system 110 of FIG. 1 may receive the subject motion data, as further described with reference to FIG. 1. The subject motion data may indicate an amplitude, a period, a bandwidth, a posture, a change of the posture, or a combination thereof. The sensor system 110 may analyze the subject motion data, or a portion thereof, to distinguish between various types of motion (e.g., the first type of motion 202 and the second type of motion 204) based on one or more of the ranges and threshold values of the table 200.

For example, a processor of the sensor system 110 may determine that the subject motion data corresponds to the first type of motion 202/202' (e.g., seizure motion) in response to determining that one or more of the range values corresponding to the first type of motion 202/202' are satisfied. As another example, the processor of the sensor system 110 may determine that the subject motion data corresponds to the second type of motion 204/204' (e.g., non-seizure motion) in response to determining that one or more of the range values corresponding to the second type of motion 204/204' are satisfied.

The processor may distinguish between the first type of motion 202 and the second type of motion 204 based on the amplitude indicated by the subject motion data and the amplitude range values 206. For example, a processor of the sensor system 110 may determine that subject motion data corresponds to the first type of motion 202 (e.g., seizure motion) in response to determining that the amplitude is within a first amplitude range 222 (e.g., 0.01 gravitational force (g) to 0.60 g) and may determine that the subject motion data corresponds to the second type of motion 204 (e.g., non-seizure motion) in response to determining that the amplitude is within a third amplitude range 226 (e.g., 0.04 g to 1.00 g). As another example, the processor may determine that the subject motion data corresponds to the first type of motion 202 in response to determining that the amplitude is within a second amplitude range 224 (e.g., 0.04 g to 0.48 g) and may determine that the subject motion data corresponds to the second type of motion 204 in response to determining that the amplitude is within a fourth amplitude range 228 (e.g., 0.48 g to 1.00 g). In a similar fashion, the processor may also distinguish between the first type of motion 202' and the second type of motion 204' based on the alternative ranges and thresholds provided in table 200'.

The processor may distinguish between the first type of motion 202 and the second type of motion 204 based on the period indicated by the subject motion data and the period threshold values 208. For example, the processor may determine that the subject motion data corresponds to the first type of motion 202 in response to determining that the period is within a first period range 230 (e.g., 100 milliseconds (ms) to 1000 ms) and may determine that the subject motion data corresponds to the second type of motion 204 in response to determining that the period is within a third period range 234 (e.g., 100 ms to 2000 ms). As another example, the processor may determine that the subject motion data corresponds to the first type of motion 202 in response to determining that the period is within a second period range 232 (e.g., 160 ms to 750 ms) and may determine that the subject motion data corresponds to the second type of motion 204 in response to determining that the period is within a fourth period range 236 (e.g., 100 ms to 1000 ms).

The processor may distinguish between the first type of motion 202 and the second type of motion 204 based on the bandwidth indicated by the subject motion data and the bandwidth threshold values 210. For example, the processor may determine that the subject motion data corresponds to the first type of motion 202 in response to determining that the bandwidth is within a first bandwidth range 240 (e.g., 0.05 to 0.60) and may determine that the subject motion data corresponds to the second type of motion 204 in response to determining that the bandwidth is within a third bandwidth range 244 (e.g., 0.00 to 0.80). As another example, the processor may determine that the subject motion data corresponds to the first type of motion 202 in response to determining that the bandwidth is within a second bandwidth range 242 (e.g., 0.10 to 0.50) and may determine that the subject motion data corresponds to the second type of motion 204 in response to determining that the bandwidth is within a fourth bandwidth range 246 (e.g., 0.10 - 0.80).

The processor may distinguish between the first type of motion 202 and the second type of motion 204 based on the posture indicated by the subject motion data and the posture threshold values 212 relative to a vertical axis 102 or some other reference system. For example, the processor may determine that the subject motion data corresponds to the first type of motion 202 in response to determining that the posture is within a first angle range 250 (e.g., less than or equal to 45 degrees) and may determine that the subject motion data corresponds to the second type of motion 204 in response to determining that the posture is greater than or equal to a third angle threshold 254 (e.g., greater than or equal to 60 degrees). As another example, the processor may determine that the subject motion data corresponds to the first type of motion 202 in response to determining that the posture is within a second angle range 252 (e.g., less than 60 degrees) and may determine that the subject motion data corresponds to the second type of motion 204 in response to determining that the posture is greater than or equal to a fourth angle range 256 (e.g., greater than 45 degrees).

The processor may distinguish between the first type of motion 202 and the second type of motion 204 based on a change of the posture indicated by the subject motion data and the change of the posture range and threshold values 214. For example, the processor may determine that the subject motion data corresponds to the first type of motion 202 in response to determining that the change of the posture over a time window is within a first change range 260 (e.g., less than 30 degrees) and may determine that the subject motion data corresponds to the second type of motion 204 in response to determining that the change of the posture of a time window is greater than a third change threshold 264 (e.g., greater than 15 degrees). As another example, the processor may determine that the subject motion data corresponds to the first type of motion 202 in response to determining that the change of the posture over a time window is within a second change range 262 (e.g., less than 20 degrees) and may determine that the subject motion data corresponds to the second type of motion 204 in response to determining that the change of the posture over a time window is greater than a fourth change threshold 266 (e.g., greater than 30 degrees).

The processor may generate an output based on identifying the first type of motion 202 or the second type of motion 204. For example, the output may indicate the type of motion identified (e.g., the first type of motion 202 or the second type of motion 204). The processor may transmit the output, the subject motion data, or both, to a base station system.

In a particular embodiment, the processor may use more than one of the threshold values of the table 200 to distinguish between the first type of motion 202 and the second type of motion 204. For example, the processor may sequentially analyze each threshold value. To illustrate, the processor may compare the amplitude to the amplitude threshold values 206 prior to comparing the period to the period threshold values 208. In this example, the processor may determine the first type of motion 202 when all the threshold values associated with the first type of motion 202 (e.g., indicated in the first column of the table 200) are satisfied. Alternatively, the processor may determine the second type of motion 204 when all the threshold values associated with the second type of motion 204 (e.g., indicated in the second column of table 200) are satisfied.

In a particular embodiment, the processor may analyze a subsequent threshold value in response to determining that an analysis of a prior threshold value is inconclusive. For example, the processor may determine that the amplitude (e.g., 0.05 g) is within a region where the first amplitude range 222 and the third amplitude range 226 overlap indicating that an analysis of the subject motion data based on the amplitude is inconclusive. In response to the determination, the processor may compare the subject motion data to the period threshold values 208 or to another threshold.

In a particular embodiment, the processor may refrain from comparing subsequent threshold values in response to determining that an analysis of a particular threshold value conclusively identifies the first type of motion 202 or the second type of motion 204. For example, the processor may determine that the amplitude is within the first amplitude range 222 and outside the third amplitude range 226 indicating that the amplitude conclusively identifies the first type of motion 202. In response to the determination, the processor may refrain from analyzing subsequent threshold values (e.g., the period threshold values, the bandwidth threshold values, the posture threshold values, the change of the posture threshold values, or a combination thereof).

Thus, one or more of the threshold values indicated by the table 200 may enable the sensor system 110 to distinguish between various types of motion based on detected subject motion data.

Referring to FIG. 3, a diagram of a particular embodiment of a system to distinguish between various types of motion is disclosed and generally designated 300. The system 300 includes a sensor system 320. The sensor system 320 may correspond to the sensor system 110 of FIG. 1. The sensor system 320 may be coupled to, or in communication with, a base station system 388 via a communication connection 384. The communication connection 384 may include a wired connection, a wireless connection, another data connection, or a combination thereof. The base station system 388 may be coupled to, or in communication with, a remote computing device 386 via a communication connection 382. The communication connection 382 may include a wired connection, a wireless connection, other data connection, or a combination thereof.

The remote computing device 386 may be a computing device that is located at a location remote from the base station system 388. For example, the remote computing device 386 may be at a location associated with a health care provider, such as a hospital. The remote computing device 386 may communicate patient information to the base station system 388, may receive motion data (e.g., the subject motion data), may receive indications of motion types (e.g., an indication of a seizure onset or offset) from the base station system 388, or a combination thereof. The remote computing device 386 may monitor the patient based on the data received from the base station system 388.

The sensor system 320 may include a preprocessor 330, a processor 340, and a memory 350. The memory 350 may be coupled to the preprocessor 330, to the processor 340, or to both. The memory 350 may include instructions that are executable by a processor (e.g., the preprocessor 330, the processor 340, or both) to operate the sensor system 320. The instructions may further cause the processor to perform one or more of the methods described herein as being performed by a sensor system (e.g., the sensor system 110 of FIG. 1). The preprocessor 330, the processor 340, or both may include one or more processors. In a particular embodiment, the preprocessor 330 may be a sensing application-specific integrated circuit (ASIC). In a particular embodiment, the processor 340 is a microprocessor (e.g., 16-bit microcontroller).

The sensor system 320 may include a user input device 360. The user input device 360 may be coupled to the preprocessor 330. The sensor system 320 may include one or more interface connectors 324. The sensor system 320 may include an input interface 302, a power manager 304, a data transfer controller 306, a battery 314, a battery protector 316, a power treatment unit 318, or a combination thereof. The input interface 302 may include a micro-universal serial bus (USB) connector. The input interface 302 may be coupled to the data transfer controller 306. The data transfer controller 306 may be coupled to the processor 340. The input interface 302 may be coupled to the power manager 304.

The power manager 304 may be coupled to the battery 314 and may control distribution of power to the sensor system 320 by the battery 314. The power manager 304 may be a USB power manager. The battery 314 may be coupled to the battery protector 316. The battery 314 may provide power to a power treatment unit 318. The power treatment unit 318 may control distribution and treatment of the power to the sensor system 320. The power treatment unit 318 may be coupled to the memory 350, the processor 340, the preprocessor 330, and the data transfer controller 306. The power treatment unit 318 may include a buck/boost converter, a boost converter, or a combination thereof.

In a particular embodiment, the sensor system 320 may include a sense amplifier 332. An input of the sense amplifier 332 may be coupled to the one or more interface connectors 324. An output of the sense amplifier 332 may be coupled to the processor 340.

The sensor system 320 may include a transceiver 346 and an antenna 348, coupled to the transceiver 346. The transceiver 346 may be coupled to the processor 340. The sensor system 320 may include a ferroelectric random-access memory (FRAM) 344, an accelerometer 342, one or more non-ECG sensors 380, an output indicator 362, or a combination thereof.

The FRAM 344 may store data and instructions for the processor 340. The FRAM 344 may perform access operations faster than access operation performed by the memory 350. The FRAM 344 may operate in the event of a power loss in the sensor system 320. The processor 340 may include, or be coupled to, the FRAM 344.

The accelerometer 342 may be a 3D accelerometer. In a particular embodiment, the accelerometer 342 may correspond to the accelerometer described with reference to FIG. 1. The accelerometer 342 may provide data (e.g., the subject motion data, as described with reference to FIG. 1), including activity and movement associated with a patient's body, to the processor 340.

The one or more other sensors 380 may be configured to sense other data. The other data may be stored in the memory 350. The other data may include heart beat data, electrical activity data generated by muscle activity or movement within the patient's body, etc.

The output indicator 362 may provide the patient (e.g., the user 108 of FIG. 1) with information associated with the sensor system 320. The information associated with the sensor system 320 may include information associated with a status of the sensor system 320, performance of the sensor system 320, operation of the sensor system 320, troubleshooting information, an indication of a type of motion detected, or a combination thereof. The information provided by the output indicator 362 may be stored in the memory 350.

The sensor system 320 may be entirely or at least partially enclosed by a housing 390. In a particular embodiment, the housing 390 may at least partially enclose the one or more interface connectors 324, the preprocessor 330, the processor 340, and the transceiver 346. The housing 390 may provide water-resistant protection for the one or more interface connectors 324, the preprocessor 330, the processor 340, and the transceiver 346.

The one or more interface connectors 324 may at least partially extend outside of the housing 390. The one or more interface connectors 324 may be operatively coupled to a connector interface of a mounting system (e.g., a patch). The mounting system may be configured to couple the housing 390 to a subject (e.g., the user 108 of FIG. 1).

The processor 340 may analyze the output received from the accelerometer 342 to distinguish between various types of motion (e.g., the first type of motion 202 and the second type of motion 204). For example, the processor 340 may analyze the subject motion data to detect a seizure event. The processor 340 may generate a particular output in response to the subject motion data indicating a particular type of motion (e.g., the first type of motion 202 or the second type of motion 204). The processor 340 may store a log indicating the detected type of motion in the memory 350.

The processor 340 may be configured to maintain a log of system activity within the sensor system 320. The log of system activity may include communication activity of the sensor system 320. The communication activity may include activation and deactivation activity performed by the transceiver 346. The log of system activity may include memory activity including operation of the memory 350, the FRAM 344, or both. The memory activity may include memory read and write operations.

The transceiver 346 may configured to communicate with one or more external devices, such as the base station system 388. The transceiver 346 may perform transmission via the antenna 348. The transceiver 346 may include a transmitter to transmit communications signals and a receiver to receive communication signals. The sensor system 320 may use the transceiver 346 to communicate with the external device via the communication connection 384. For example, the sensor system 320 may transmit motion data (e.g., the subject motion data), an output indicating a detect type of motion (e.g., the first type of motion 202 or the second type of motion 204), or both, via transmission from the transmitter to the base station system 388. The communication connection 384 may facilitate data communication according to one or more of wireless mobile data communication compliant standards including code division multiple access (CDMA), time division multiple access (TDMA), frequency division multiple access (FDMA), orthogonal frequency division multiple access (OFDMA), single-carrier frequency division multiple access (SC-FDMA), a global system for mobile communications (GSM), enhanced data rates for GSM evolution (EDGE), evolved EDGE, Universal Mobile Telecommunications System (UMTS), Worldwide Interoperability for Microwave Access (Wi-Max), general packet radio service (GPRS), 3rd generation partnership project (3GPP), 3GPP2, 4th generation (4G), long term evolution (LTE), 4G-LTE, high speed packet access (HSPA), HSPA+, Institute of Electrical and Electronics Engineers (IEEE) 802.11x, or a combination thereof.

The user input device 360 may enable the patient to provide input to the sensor system 320. The input may be used to control operation of the sensor system 320. For example, the user input device 360 may be configured to cause the processor 340 to process the subject motion data in response to user input via the user input device 360.

The system 300 may operable to distinguish between various types of motion and to store information regarding a detected type of motion. The information may be communicated to a user (e.g., the user 108), to the base station system 388, to the remote computing device 386, or a combination thereof. The information may be used to log and monitor user activity. For example, the information may be used to monitor a frequency of seizures experienced by the user 108. The information may facilitate medical diagnostics and treatment of the user 108.

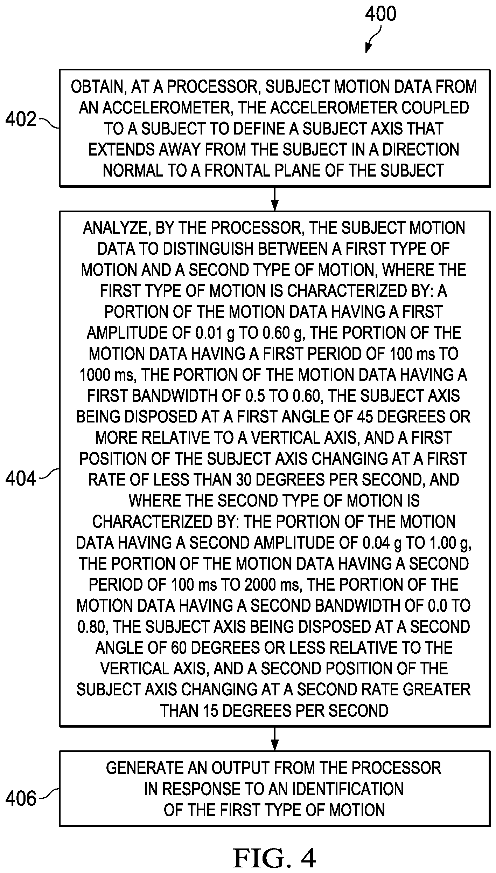

FIG. 4 is flow chart of a particular embodiment of a method 400 that may be performed at a sensor system. For example, the method 400 may be performed by the sensor system 110 of FIG. 1, the sensor system 320 of FIG. 3, or both.

The method 400 includes obtaining, at a processor, subject motion data from an accelerometer, at 402. The accelerometer may be coupled to a subject to define a subject axis that extends away from the subject in a direction normal to a frontal plane of the subject. For example, a processor of the sensor system 110 of FIG. 1 may receive subject motion data from an accelerometer, as further described with reference to FIG. 1. The accelerometer may be coupled to a subject (e.g., the user 108 of FIG. 1) to define a subject axis, as further described with reference to FIG. 1.

The method 400 also includes analyzing, by the processor, the subject motion data to distinguish between a first type of motion and a second type of motion, at 404. For example, a processor of the sensor system 110 of FIG. 1 may analyze the subject motion data to distinguish between the first type of motion 202 and the second type of motion 204, as further described with reference to FIG. 2A.

The first type of motion may be characterized by a portion of the motion data having a first amplitude of 0.01 g to 0.60 g, the portion of the motion data having a first period of 100 ms to 1000 ms, the portion of the motion data having a first bandwidth of 0.05 to 0.60, the subject axis being disposed at a first angle of 45 degrees or more relative to a vertical axis, a first position of the subject axis changing less than 30 degrees over a time window, or a combination thereof. The second type of motion may be characterized by the portion of the motion data having a second amplitude of 0.04 g to 1.00 g, the portion of the motion data having a second period of 100 ms to 2000 ms, the portion of the motion data having a second bandwidth of 0.00 to 0.80, the subject axis being disposed at a second angle of 60 degrees or less relative to the vertical axis, a second position of the subject axis changing greater than 15 degrees over a time window, or a combination thereof.

In a particular embodiment, the first type of motion may be characterized by a portion of the motion data having a first amplitude of 0.04 g to 0.48 g, the portion of the motion data having a first period of 160 ms to 750 ms, the portion of the motion data having a first bandwidth of 0.10 to 0.50, the subject axis being disposed at a first angle of greater than 60 degrees relative to a vertical axis, a first position of the subject axis changing at less than 20 degrees over a time window, or a combination thereof. The second type of motion may be characterized by the portion of the motion data having a second amplitude of 0.48 g to 1.00 g, the portion of the motion data having a second period of 100 ms to 1000 ms, the portion of the motion data having a second bandwidth of 0.10 to 0.80, the subject axis being disposed at a second angle of less than 45 degrees relative to the vertical axis, a second position of the subject axis changing at greater than 30 degrees over a time window, or a combination thereof.

The method 400 further includes generating an output from the processor in response to an identification of the first type of motion, at 406. For example, a processor of the sensor system 110 of FIG. 1 may generate an output in response to an identification of the first type of motion 202, as further described with reference to FIG. 2A.

With reference to FIGS. 5-10, another embodiment of the sensor system 110 is provided that may include similar or the same components describe above in other embodiments. FIG. 5 illustrates components of a motion sensor system similar to the sensor system described with regard to FIGS. 1A-1B. FIG. 5 illustrates a motion monitoring system 500 that includes a sensor-patch 501 having a sensor 502 coupled to a patch 504. The sensor-patch 501 is configured to adhere to the skin of the subject, with the patch 504 having an adhesive surface that couples to the subject's skin. The patch 504 also includes a coupling supporting the sensor 502. The sensor 502 is removable from the patch 504, and the patch 504 provides an interface between the sensor 504 and the subject. FIG. 5 also illustrates a hub 506 that is configured to interface and communicate with the sensor 502 to send and receive information and, in particular, to receive information obtained by the sensor 502 via the patch 504. As also illustrated in FIG. 5, the hub 506 includes a visual display 508 providing information regarding the connectivity with the sensor 502 and providing an interface allowing a user to communicate with and program the operation of the sensor system 110 and, in particular, the sensor 502. The hub 506 also includes controls 510 and indicators 512 that allow the user or subject to enter information or receive information regarding the operation of the sensor system 110. FIG. 5 also illustrates a communication device 514 which can be a smart phone configured to communicate or otherwise interface with the sensor 502 and/or the hub 506. As can be appreciated, the communication device can be configured as an app operating on a smart phone, and can be configured to be used by a caregiver or the subject.

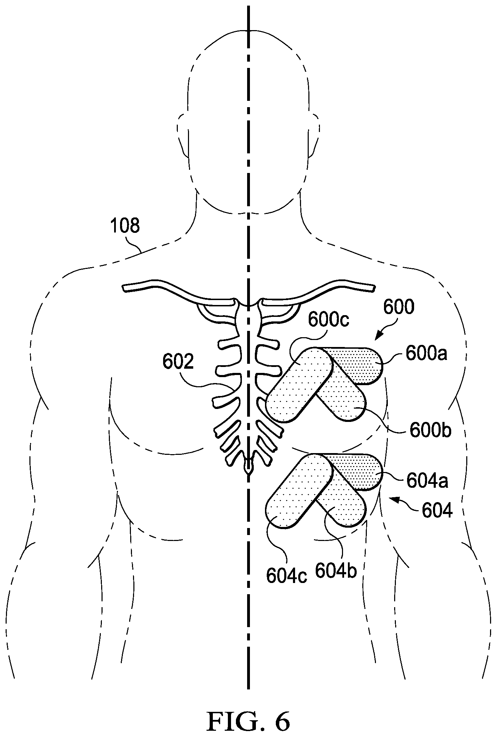

FIG. 6 illustrates various techniques for connecting the sensor-patch 501 to the subject 108. As illustrated, the sensor-patch 501 and, more particularly, the patch 504 may be applied to the subject on the chest or over the subject's rib cage at several locations. A first location 600 is in line with the subject's sternum 602. A second location 604 is provided below the sternum 602. As illustrated, the patch 504 may be disposed at the first location 600 or the second location 604 at different orientations. For example, the patch 504 may be disposed in horizontal orientation 600a or 604a. In another example, the patch 504 may be disposed at an angled orientation 600b, 600c, 604b, or 604c. Preferably, the sensor-patch 501 is disposed to a side of the sternum 604. The sensor-patch may be disposed on the chest of the subject or on the subject's back. As can be appreciated, the various positions and orientations of the sensor-patch 501 may be evaluated for a particular subject or to account for subject preferences or environmental factors. Preferably, the sensor-patch 501 is disposed in a location that allows the sensor 502 to acquire a heart signal from the subject as well as a motion signal.

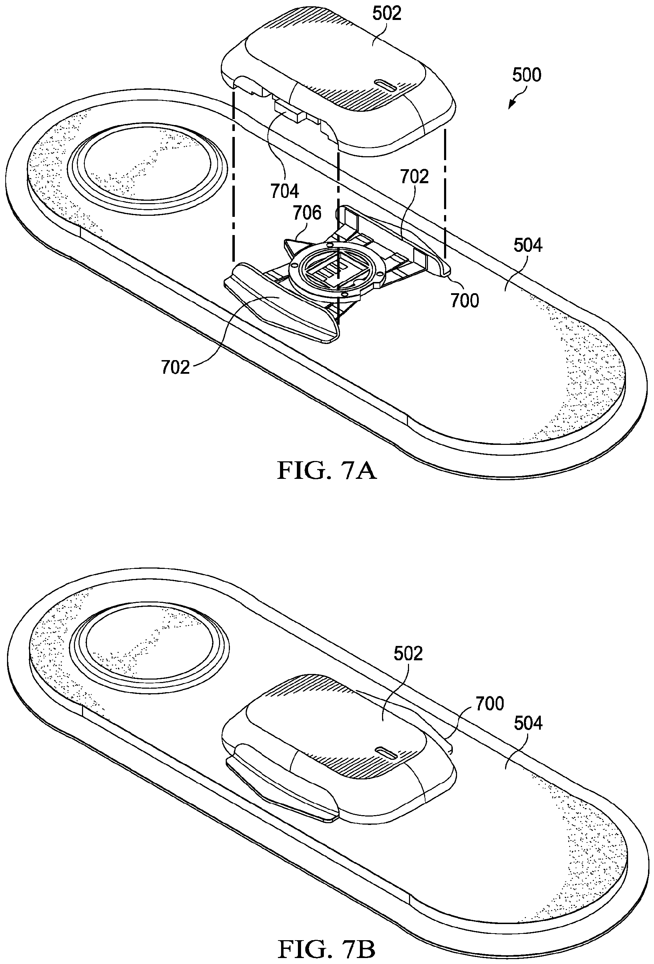

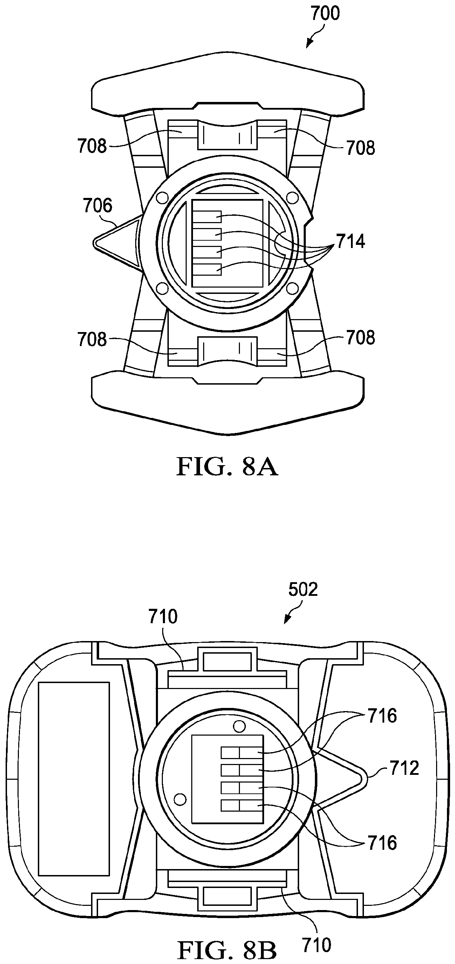

FIG. 7 illustrates another view of the sensor-patch 501 of FIG. 5 but with the sensor 502 disconnected from the patch 504. As illustrated, the patch 504 includes a mounting bracket 700 configured to couple a bracket guide 702 with a mating portion 704 of the sensor 502. The mounting bracket 700 may include a key 706 that requires the sensor 502 to be coupled to the mounting bracket 700 in a single configuration. FIG. 7B illustrates the sensor-patch of FIG. 7A after the sensor 502 and patch 504 are coupled via the interface provided by the mounting bracket 700. FIGS. 8A and 8B provide complimentary views of the mating surfaces of the sensor 502 and the mounting bracket 700. FIG. 8A illustrates the mounting bracket 700 in greater detail, showing snaps 708 that engage with the mating structure 710 on the sensor 502 illustrated on FIG 8B. Also illustrated on FIG. 8B is a key recess 712 configured to receive the key 706. Also illustrated in FIG 8A are patch terminals 714 and illustrated in FIG. 8B are sensor terminals 716 that engage each other to provide connectivity between the sensor 502 and the battery and internal components of the patch 504.

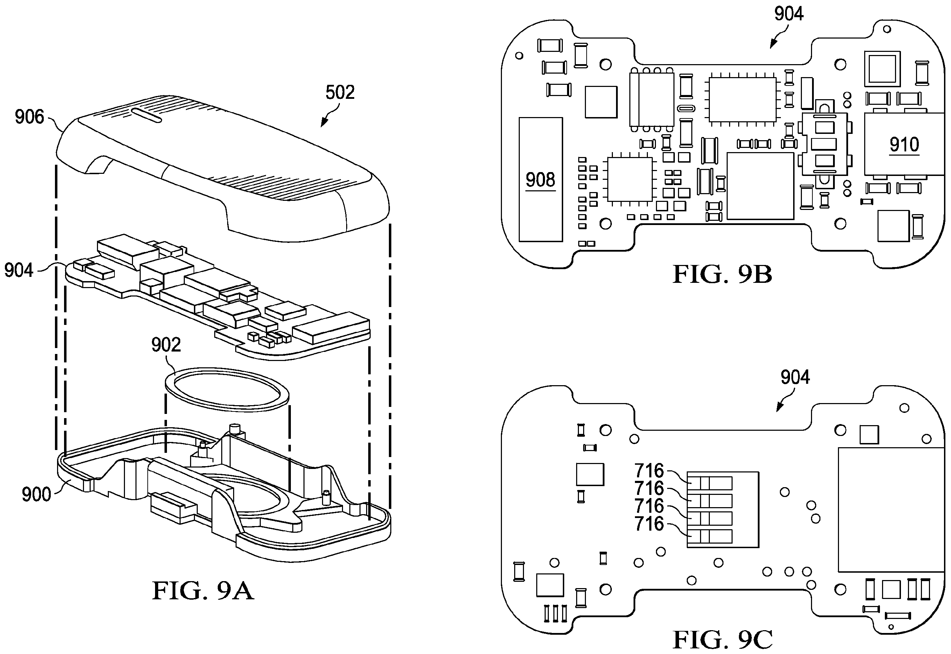

FIG. 9A illustrates an exploded view of the sensor 502 showing a bottom cover 900, a seal 902, a printed circuit board 904, and a top cover 906. Although not shown, the printed circuit board 904 supports three accelerometers disposed to define x, y, and z axes. FIG. 9B illustrates a top view of the circuit board 904 and FIG. 9C illustrates a bottom view of the same circuit board 904, showing an antenna 908, a micro HDMI 910, and the sensor terminals 716.

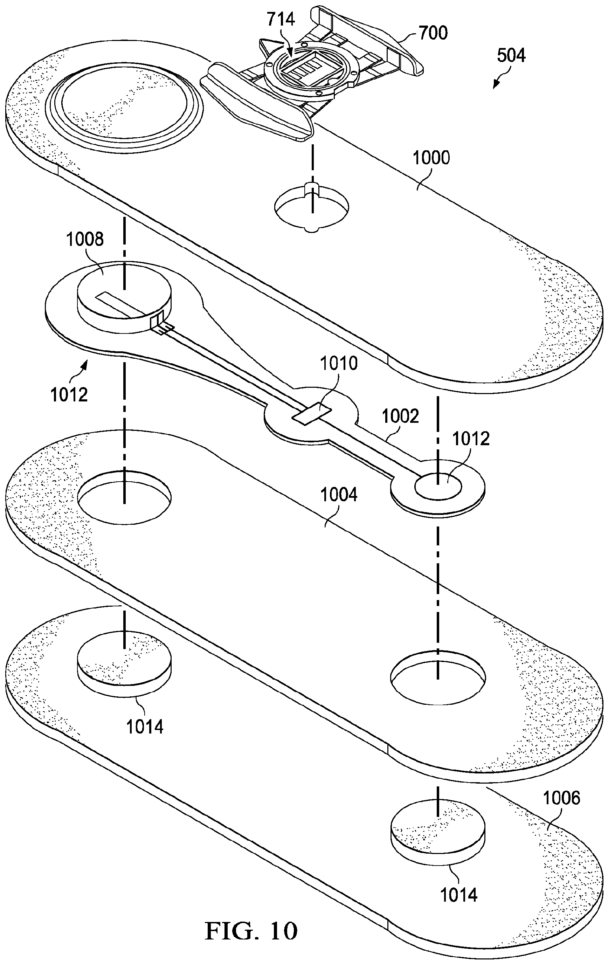

FIG. 10 illustrates an exploded view of the patch 504 showing the mounting bracket 700, a top cover 1000 supporting the mounting bracket 700, a flex circuit 1002, an insulating later 1004, and an adhesive layer 1006. The flex circuit 1002 supports a battery 1008 connected to terminals 1010 with the terminals 1010 positioned to connect to the patch terminal 714 to supply power to the sensor 502 when assembled. The flex circuit 1002 also supports two electrodes 1012, with one shown in FIG. 10 and the other disposed under the battery 1008. The electrodes 1012 are also connected to the terminals 1010 so allow the sensor 502 to interface with the electrodes 1012 when assembled. The adhesive layer 1006 is configured to adhere to the skin of the subject and to provide two hydrogels 1014 that enhance the connectivity between the electrodes 1012 and the subject's skin.

FIG. 11 illustrates a signal 1100 representing subject motion data received from a motion monitoring device such as those illustrated in FIGS. 1A-1B and 5-10. As illustrated, the signal 1110 includes an amplitude signal 1102, a period signal 1104, a bandwidth signal 1106, a position signal 1108, and a change-in-position signal 1110 that include various components of the subject motion data as described above.

As can be appreciated from the embodiment illustrated in FIGS. 5-10, a two-part sensor-patch 501 is provided that advantageously allows the sensor 502 containing the accelerometers and other reusable components to be disconnected from the patch 504 supporting disposable components such as the battery 1008, the adhesive layer 1006, and the hydrogels 1014. The two-part sensor-patch 501 also advantageously allows the use or subject to disconnect soiled components that may be found in the patch 504 from clean components that may be found in the sensor 502.

Although the description above contains many specificities, these specificities are utilized to illustrate some particular embodiments of the disclosure and should not be construed as limiting the scope of the disclosure. The scope of this disclosure should be determined by the claims and their legal equivalents. A method or device does not have to address each and every problem to be encompassed by the present disclosure. All structural, chemical and functional equivalents to the elements of the disclosure that are known to those of ordinary skill in the art are expressly incorporated herein by reference and are intended to be encompassed by the present claims. A reference to an element in the singular is not intended to mean one and only one, unless explicitly so stated, but rather it should be construed to mean at least one. No claim element herein is to be construed under the provisions of 35 U.S.C. § 112, sixth paragraph, unless the element is expressly recited using the phrase "means for." Furthermore, no element, component or method step in the present disclosure is intended to be dedicated to the public, regardless of whether the element, component or method step is explicitly recited in the claims.

The disclosure is described above with reference to drawings. These drawings illustrate certain details of specific embodiments of the systems and methods and programs of the present disclosure. However, describing the disclosure with drawings should not be construed as imposing on the disclosure any limitations that may be present in the drawings. The present disclosure contemplates methods, systems and program products on any machine-readable media for accomplishing its operations. The embodiments of the present disclosure may be implemented using an existing computer processor, a special purpose computer processor, or by a hardwired system.

As noted above, embodiments within the scope of the present disclosure include program products including machine-readable media for carrying or having machine-executable instructions or data structures stored thereon. Such machine-readable media can be any available media which can be accessed by a general purpose or special purpose computer or other machine with a processor. By way of example, such machine-readable media can include RAM, ROM, EPROM, EEPROM, CD ROM or other optical disk storage, magnetic disk storage or other magnetic storage devices, or any other medium which can be used to carry or store program code in the form of machine-executable instructions or data structures and which can be accessed by a general purpose or special purpose computer or other machine with a processor. The disclosure may be utilized in a non-transitory media. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general purpose computer, a special purpose computer, or special purpose processing machines to perform a certain function or group of functions.

Embodiments of the disclosure are described in the general context of method steps which may be implemented in one embodiment by a program product including machine-executable instructions, such as program code, for example, in the form of program modules executed by machines in networked environments. Generally, program modules include routines, programs, objects, components, data structures, etc., that perform particular tasks or implement particular abstract data types. Machine-executable instructions, associated data structures, and program modules represent examples of program code for executing steps of the methods disclosed herein. The particular sequence of such executable instructions or associated data structures represent examples of corresponding acts for implementing the functions described in such steps

Embodiments of the present disclosure may be practiced in a networked environment using logical connections to one or more remote computers having processors. Logical connections may include a local area network (LAN) and a wide area network (WAN) that are presented here by way of example and not limitation. Such networking environments are commonplace in office-wide or enterprise-wide computer networks, intranets and the Internet and may use a wide variety of different communication protocols. Those skilled in the art will appreciate that such network computing environments will typically encompass many types of computer system configurations, including personal computers, hand-held devices, multiprocessor systems, microprocessor-based or programmable consumer electronics, network PCs, servers, minicomputers, mainframe computers, and the like. For example, the network computing environment may include the sensor system 110 of FIG. 1, the system 300, the sensor system 320, the base station system 388, the remote computing device 386 of FIG.3, or any combination thereof. Embodiments of the disclosure may also be practiced in distributed computing environments where tasks are performed by local and remote processing devices that are linked (either by hardwired links, wireless links, or by a combination of hardwired or wireless links) through a communications network. In a distributed computing environment, program modules may be located in both local and remote memory storage devices. For example, the sensor system 320 may detect motion data (e.g., the subject motion data) and may send the motion data to the base station system 388, the remote computing device 386, or both. The base station system 388, the remote computing device 386, or both, may distinguish between various types of motion (e.g., the first type of motion 202 and the second type of motion 204) based on the motion data

An exemplary system for implementing the overall system or portions of the disclosure might include a general purpose computing device in the form of a computer, including a processing unit, a system memory, and a system bus that couples various system components including the system memory to the processing unit. For example, the general purpose computing device may include the sensor system 110 of FIG. 1, the system 300, the sensor system 320, the base station system 388, the remote computing device 386 of FIG.3, or any combination thereof. The system memory may include read only memory (ROM) and random access memory (RAM). The computer may also include a magnetic hard disk drive for reading from and writing to a magnetic hard disk, a magnetic disk drive for reading from or writing to a removable magnetic disk, and an optical disk drive for reading from or writing to a removable optical disk such as a CD ROM or other optical media. The drives and their associated machine-readable media provide nonvolatile storage of machine-executable instructions, data structures, program modules, and other data for the computer.

It should be noted that although the flowcharts provided herein show a specific order of method steps, it is understood that the order of these steps may differ from what is depicted. Also two or more steps may be performed concurrently or with partial concurrence. Such variation will depend on the software and hardware systems chosen and on designer choice. It is understood that all such variations are within the scope of the disclosure.

The foregoing description of embodiments of the disclosure has been presented for purposes of illustration and description. It is not intended to be exhaustive or to limit the disclosure to the precise form disclosed, and modifications and variations are possible in light of the above teachings or may be acquired from practice of the disclosure. The embodiments were chosen and described in order to explain the principals of the disclosure and its practical application to enable one skilled in the art to utilize the disclosure in various embodiments and with various modifications as are suited to the particular use contemplated.

The Abstract of the Disclosure is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, the claimed subject matter may be directed to less than all of the features of any of the disclosed embodiments.

## Claims

1. A method of distinguishing between a first type of motion and a second type of motion of a subject **characterized by** a signal corresponding to the first and second types of motions, the method comprising:
receiving the signal at a processor, the signal being representative of subject motion data of the subject, the subject motion data including subject position data and subject change-in-position data;
analyzing, by the processor, the subject motion data to distinguish between the first type of motion occurring over a first time period and the second type of motion occurring over a second time period,
wherein the first type of motion is **characterized by**:
a first bandwidth that is inclusively within a first bandwidth range,
the subject position data indicating that the subject is in a recumbent orientation throughout the first time period, the recumbent orientation defined by an initial calibration during which the subject is in the recumbent position while defining an offset angle between a subject axis extending from the subject and a vertical axis, the recumbent orientation further defined by the subject axis remaining inclusively within an offset angle range throughout the first time period, and
the subject change-in-position data indicating that a first rotation parameter of the subject change-in-position data is inclusively within a rotation range throughout the first time period, and
wherein the second type of motion is **characterized by**:
a second bandwidth that is inclusively within a second bandwidth range,
the subject position data indicating that the subject is in an upright orientation throughout the second time period, the upright orientation defined by the offset angle equaling or exceeding an offset angle threshold throughout the second time period, and
the subject change-in-position data indicating that a second rotation parameter of the subject change-in-position data is greater than a rotation threshold throughout the second time period; and
generating a first output from the processor in response to an identification of the first type of motion and generating a second output from the processor in response to an identification of the second type of motion, the first output being an affirmation of a seizure condition of the subject and the second output being at least one of a null output and an affirmation of a non-seizure condition of the subject; and
wherein the subject motion data is further **characterized by** at least one of the following:
a first period that is at a minimum and/or a maximum of a first period range for at least a portion of the first time period,
the first bandwidth is at a minimum and/or a maximum of a first bandwidth range for at least a portion of the first time period,
the offset angle is at a minimum and/or a maximum of an offset angle range for at least a portion of the first time period,
the first rotation parameter is at a minimum and/or a maximum of a rotation range for at least a portion of first time period,a second period that is at a minimum and/or a maximum of a second period range for at least a portion of the second time period, or
the second bandwidth is at a minimum and/or a maximum of a second bandwidth range for at least a portion of the second time period.

2. The method of claim 1 wherein,
the first type of motion is further **characterized by**:
a first amplitude that is inclusively within a first amplitude range, and
the second type of motion is further **characterized by**:
a second amplitude that is inclusively within a second amplitude range.

3. The method of claim 1 wherein the signal is provided by an accelerometer coupled to the subject and disposed to acquire the subject motion data.

4. The method of claim 3 wherein the accelerometer defines a subject axis extending away from the subject to define a baseline orientation of the subject, the recumbent position and the upright orientation each being indicated by reference to the baseline orientation.

5. The method of claim 1 wherein the signal is provided by a visualization device that acquires an image of the subject, the image of the subject corresponding to the subject motion data.

6. The method of claim 5 wherein the visualization device acquires the image of the subject via at least one of a video device or a thermal imaging system and at least one of a motion detector, a depth sensor, or an infrared laser device.

7. A motion monitoring system for monitoring a motion of a subject, the motion monitoring system comprising:
a housing;
a mounting system configured to couple the housing to the subject;
an accelerometer disposed on the housing, the accelerometer configured to obtain subject motion data, the subject motion data including subject position data and subject change-in-position data; and
a processor configured to analyze the subject motion data to distinguish between a first type of motion occurring over a first time period and a second type of motion occurring over a second time period,
wherein the first type of motion is **characterized by**:
a first bandwidth that is inclusively within a first bandwidth range,
the subject position data indicating that the subject is in a recumbent orientation throughout the first time period, the recumbent orientation defined by an initial calibration during which the subject is in the recumbent position while defining an offset angle between a subject axis extending from the subject and a vertical axis, the recumbent orientation further defined by the subject axis remaining inclusively within an offset angle range throughout the first time period, and
the subject change-in-position data indicating that a first rotation parameter of the subject change-in-position data is inclusively within a rotation range throughout the first time period, and
wherein the second type of motion is **characterized by**:
a second bandwidth that is inclusively within a second bandwidth range,
the subject position data indicating that the subject is in an upright orientation throughout the second time period, the upright orientation defined by the offset angle equaling or exceeding an offset angle threshold throughout the second time period, and
the subject change-in-position data indicating that a second rotation parameter of the subject change-in-position data is greater than a rotation threshold throughout the second time period; and
an interface responsive to the processor, the interface providing a first output from the processor in response to an identification of the first type of motion and
providing a second output from the processor in response to an identification of the second type of motion, the first output being an affirmation of a seizure condition of the subject and the second output being at least one of a null output and an affirmation of a non-seizure condition of the subject; and
wherein the subject motion data is further **characterized by** at least one of the following:
a first period that is at a minimum and/or a maximum of a first period range for at least a portion of the first time period,
the first bandwidth is at a minimum and/or a maximum of a first bandwidth range for at least a portion of the first time period,
the offset angle is at a minimum and/or a maximum of a offset angle range for at least a portion of the first time period,
the first rotation parameter is at a minimum and/or a maximum of a rotation range for at least a portion of first time period,a second period that is at a minimum and/or a maximum of the second period range for at least a portion of the second time period, or
the second bandwidth is at a minimum and/or a maximum of the second bandwidth range for at least a portion of the second time period.

8. The motion monitoring system of claim 7 wherein,
the first type of motion is further **characterized by**:
a first amplitude that is inclusively within a first amplitude range, and
the second type of motion is further **characterized by**:
a second amplitude that is inclusively within a second amplitude range.

9. The motion monitoring system of claim 7 wherein the accelerometer defines a subject axis extending away from the subject to define a baseline orientation of the subject, the recumbent position and the upright orientation each being indicated by reference to the baseline orientation.

## Patentansprüche

1. Ein Verfahren zum Unterscheiden zwischen einer ersten Art einer Bewegung und einer zweiten Art einer Bewegung eines Subjekts, charakterisiert durch ein Signal entsprechend der ersten Art und der zweiten Art einer Bewegung, wobei das Verfahren umfasst:
Empfangen des Signals an einem Prozessor, wobei das Signal für Subjektbewegungsdaten des Subjekts repräsentativ ist, wobei die Subjektbewegungsdaten Subjektpositionsdaten und Subjektpositionsänderungsdaten einschließen;
Analysieren der Subjektbewegungsdaten durch den Prozessor, um zwischen der ersten Art einer Bewegung, die über eine erste Zeitdauer auftritt, und der zweiten Art einer Bewegung, die über eine zweite Zeitdauer auftritt, zu unterscheiden,
wobei die erste Art einer Bewegung charakterisiert ist:
durch eine erste Bandbreite, die innerhalb eines ersten Bandbreitenbereichs liegt,
dadurch, dass die Subjektpositionsdaten anzeigen, dass das Subjekt sich über die gesamte erste Zeitdauer in einer liegenden Orientierung befindet, wobei die liegende Orientierung durch eine anfängliche Kalibration definiert ist, während der sich das Subjekt in einer liegenden Orientierung befindet, wobei ein Abweichungswinkel zwischen einer Subjektachse, die sich von dem Subjekt erstreckt, und einer vertikalen Achse definiert ist, wobei die liegende Orientierung weiterhin dadurch definiert ist, dass die Subjektachse über die gesamte erste Zeitdauer innerhalb eines Abweichungswinkelbereichs verbleibt, und
dadurch, dass die Subjektpositionsänderungsdaten anzeigen, dass ein erster Rotationsparameter der Subjektpositionsänderungsdaten über die gesamte erste Zeitdauer innerhalb eines Rotationsbereichs liegt, und
wobei die zweite Art einer Bewegung charakterisiert ist:
durch eine zweite Bandbreite, die innerhalb eines zweiten Bandbreitenbereichs liegt,
dadurch, dass die Subjektpositionsdaten anzeigen, dass das Subjekt sich über die gesamte zweite Zeitdauer in einer aufrechten Orientierung befindet, wobei die aufrechte Orientierung durch einen Abweichungswinkel definiert ist, der über die gesamte zweite Zeitdauer gleich einem Abweichungswinkelschwellwert ist oder diesen überschreitet, und
dadurch, dass die Subjektpositionsänderungsdaten anzeigen, dass ein zweiter Rotationsparameter der Subjektpositionsänderungsdaten über die gesamte zweite Zeitdauer größer als ein Rotationsschwellwert ist; und
Erzeugen einer ersten Ausgabe des Prozessors in Reaktion auf eine Identifikation der ersten Art einer Bewegung und Erzeugen einer zweiten Ausgabe des Prozessors in Reaktion auf eine Identifikation der zweiten Art einer Bewegung, wobei die erste Ausgabe eine Bestätigung eines Anfallszustands des Subjekts ist und die zweite Ausgabe zumindest eines von einer Nullausgabe und einer Bestätigung eines Nicht-Anfallszustand des Subjekts ist; und
wobei die Subjektbewegungsdaten weiterhin durch zumindest eines von dem Folgenden charakterisiert sind:
eine erste Zeitdauer, die an einem Minimum und/oder einem Maximum eines ersten Zeitdauerbereichs über zumindest einen Teil der ersten Zeitdauer liegt, dadurch, dass die erste Bandbreite an einem Minimum und/oder Maximum eines ersten Bandbreitenbereichs über zumindest einen Teil der ersten Zeitdauer liegt,
dadurch, dass der Abweichungswinkel an einem Minimum und/oder Maximum eines Abweichungswinkelbereichs über zumindest einen Teil der ersten Zeitdauer liegt,
dadurch, dass der erste Rotationsparameter an einem Minimum und/oder Maximum eines Rotationsbereichs über zumindest einen Teil der ersten Zeitdauer liegt,
eine zweite Zeitdauer, die an einem Minimum und/oder einem Maximum eines zweiten Zeitdauerbereichs über zumindest einen Teil der zweiten Zeitdauer liegt, oder
dadurch, dass die zweite Bandbreite an einem Minimum und/oder Maximum eines zweiten Bandbreitenbereichs über zumindest einen Teil der zweiten Zeitdauer liegt.

2. Das Verfahren von Anspruch 1, in dem die erste Art einer Bewegung weiterhin charakterisiert ist durch:
eine erste Amplitude die innerhalb eines ersten Amplitudenbereichs liegt, und
die zweite Art einer Bewegung weiterhin charakterisiert ist durch:
eine zweite Amplitude die innerhalb eines zweiten Amplitudenbereichs liegt.

3. Das Verfahren von Anspruch 1, in dem das Signal von einem Beschleunigungsmesser bereitgestellt wird, der mit dem Subjekt verbunden ist und so angeordnet ist, dass er die Subjektbewegungsdaten erfasst.

4. Das Verfahren von Anspruch 3, in dem der Beschleunigungsmesser eine Subjektachse definiert, die sich von dem Subjekt erstreckt, um eine Ausgangslageorientierung des Subjekts zu definieren, wobei die liegende Position und die aufrechte Orientierung mit Bezug auf die Ausgangslageorientierung angezeigt werden.

5. Das Verfahren von Anspruch 1, in dem das Signal von einer Visualisierungseinrichtung bereitgestellt wird, die ein Bild des Subjekts aufnimmt, wobei das Bild des Subjekts den Subjektbewegungsdaten entspricht.

6. Das Verfahren von Anspruch 5, in dem die Visualisierungseinrichtung das Bild des Subjekts durch zumindest eines von einer Videoeinrichtung oder einem thermischen Bildgebungssystem und zumindest eines von einem Bewegungsdetektor, einem Tiefensensor oder einer Infrarotlasereinrichtung aufnimmt.

7. Ein Bewegungsüberwachungssystem zum Überwachen einer Bewegung eines Subjekts, wobei das Bewegungsüberwachungssystem umfasst:
ein Gehäuse;
ein Befestigungssystem das dazu ausgebildet ist, das Gehäuse an dem Subjekt zu befestigen;
ein Beschleunigungsmesser, der auf dem Gehäuse angeordnet ist, wobei der Beschleunigungsmesser dazu ausgebildet ist, Subjektbewegungsdaten zu gewinnen, wobei die Subjektbewegungsdaten Subjektpositionsdaten und Subjektpositionsänderungsdaten einschließen; und
einen Prozessor, der dazu ausgebildet ist, die Subjektbewegungsdaten zu analysieren, um zwischen einer ersten Art einer Bewegung, die über eine erste Zeitdauer auftritt, und einer zweiten Art einer Bewegung, die über eine zweite Zeitdauer auftritt, zu unterscheiden,
wobei die erste Art einer Bewegung charakterisiert ist:
durch eine erste Bandbreite, die innerhalb eines ersten Bandbreitenbereichs liegt, dadurch, dass die Subjektpositionsdaten anzeigen, dass das Subjekt sich über die gesamte erste Zeitdauer in einer liegenden Orientierung befindet, wobei die liegende Orientierung durch eine anfängliche Kalibration definiert ist, während der sich das Subjekt in einer liegenden Orientierung befindet, wobei ein Abweichungswinkel zwischen einer Subjektachse, die sich von dem Subjekt erstreckt, und einer vertikalen Achse definiert ist, wobei die liegende Orientierung weiterhin dadurch definiert ist, dass die Subjektachse über die gesamte erste Zeitdauer innerhalb eines Abweichungswinkelbereichs verbleibt, und
dadurch, dass die Subjektpositionsänderungsdaten anzeigen, dass ein erster Rotationsparameter der Subjektpositionsänderungsdaten über die gesamte erste Zeitdauer innerhalb eines Rotationsbereichs liegt, und
wobei die zweite Art einer Bewegung charakterisiert ist:
durch eine zweite Bandbreite, die innerhalb eines zweiten Bandbreitenbereichs liegt,
dadurch, dass die Subjektpositionsdaten anzeigen, dass das Subjekt sich über die gesamte zweite Zeitdauer in einer aufrechten Orientierung befindet, wobei die aufrechte Orientierung durch einen Abweichungswinkel definiert ist, der über die gesamte zweite Zeitdauer gleich einem Abweichungswinkelschwellwert ist oder diesen überschreitet, und
dadurch, dass die Subjektpositionsänderungsdaten anzeigen, dass ein zweiter Rotationsparameter der Subjektpositionsänderungsdaten über die gesamte zweite Zeitdauer größer als ein Rotationsschwellwert ist; und
eine Schnittstelle, die von dem Prozessor bedarfsgesteuert wird, wobei die Schnittstelle eine erste Ausgabe des Prozessors in Reaktion auf eine Identifikation der ersten Art einer Bewegung und eine zweite Ausgabe des Prozessors in Reaktion auf eine Identifikation der zweiten Art einer Bewegung bereitstellt, wobei die erste Ausgabe eine Bestätigung eines Anfallszustands des Subjekts ist und die zweite Ausgabe zumindest eines von einer Nullausgabe und einer Bestätigung eines Nicht-Anfallszustand des Subjekts ist; und
wobei die Subjektbewegungsdaten weiterhin durch zumindest eines von dem Folgenden charakterisiert sind:
eine erste Zeitdauer, die an einem Minimum und/oder einem Maximum eines ersten Zeitdauerbereichs über zumindest einen Teil der ersten Zeitdauer liegt,
dadurch, dass die erste Bandbreite an einem Minimum und/oder Maximum eines ersten Bandbreitenbereichs über zumindest einen Teil der ersten Zeitdauer liegt,
dadurch, dass der Abweichungswinkel an einem Minimum und/oder Maximum eines Abweichungswinkelbereichs über zumindest einen Teil der ersten Zeitdauer liegt,
dadurch, dass der erste Rotationsparameter an einem Minimum und/oder Maximum eines Rotationsbereichs über zumindest einen Teil der ersten Zeitdauer liegt,
eine zweite Zeitdauer, die an einem Minimum und/oder einem Maximum eines zweiten Zeitdauerbereichs über zumindest einen Teil der zweiten Zeitdauer liegt, oder
dadurch, dass die zweite Bandbreite an einem Minimum und/oder Maximum eines zweiten Bandbreitenbereichs über zumindest einen Teil der zweiten Zeitdauer liegt.

8. Das Bewegungsüberwachungssystem von Anspruch 7, in dem die erste Art einer Bewegung weiterhin charakterisiert ist durch:
eine erste Amplitude die innerhalb eines ersten Amplitudenbereichs liegt, und
die zweite Art einer Bewegung weiterhin charakterisiert ist durch:
eine zweite Amplitude die innerhalb eines zweiten Amplitudenbereichs liegt.

9. Das Bewegungsüberwachungssystem von Anspruch 7, in dem der Beschleunigungsmesser eine Subjektachse definiert, die sich von dem Subjekt erstreckt, um eine Ausgangslageorientierung des Subjekts zu definieren, wobei die liegende Position und die aufrechte Orientierung mit Bezug auf die Ausgangslageorientierung angezeigt werden.

## Revendications

1. Procédé destiné à effectuer une distinction entre un premier type de mouvement et un second type de mouvement d'un sujet **caractérisé par** un signal correspondant au premier et au second type de mouvement, le procédé comprenant :
la réception du signal au niveau d'un processeur, le signal étant représentatif des données de mouvement de sujet du sujet, les données de mouvement de sujet comprenant les données de position de sujet et les données de changement de position du sujet ;
l'analyse, par le processeur, des données de mouvement de sujet pour effectuer une distinction entre le premier type de mouvement survenant sur une première période de temps et le second type de mouvement survenant sur une seconde période de temps,
le premier type de mouvement étant **caractérisé par** :
une première largeur de bande qui se trouve inclusivement à l'intérieur d'une première plage de largeurs de bande,
les données de position de sujet indiquant que le sujet se trouve dans une orientation couchée à travers la première période de temps, l'orientation couchée définie par un étalonnage initial durant lequel le sujet se trouve en position couchée tout en définissant un angle décalé entre un axe de sujet s'étendant depuis le sujet et un axe vertical, l'orientation couchée définie en outre par l'axe de sujet restant inclusivement à l'intérieur d'une plage d'angles décalés à travers la première période de temps, et
les données de changement de position du sujet indiquant qu'un premier paramètre de rotation des données de changement de position du sujet se trouve inclusivement à l'intérieur d'une plage de rotations à travers la première période de temps, et
le second type de mouvement étant **caractérisé par** :
une seconde largeur de bande qui se trouve inclusivement à l'intérieur d'une seconde plage de largeurs de bande,
les données de position de sujet indiquant que le sujet se trouve dans une orientation verticale à travers la seconde période de temps, l'orientation verticale définie par l'angle décalé qui est égal ou qui excède un seuil d'angle décalé à travers la seconde période de temps, et
les données de changement de position du sujet indiquant qu'un second paramètre de rotation des données de changement de position du sujet est supérieur à un seuil de rotation à travers la seconde période de temps ; et
la génération d'une première sortie depuis le processeur en réponse à une identification du premier type de mouvement et la génération d'une seconde sortie depuis le processeur en réponse à une identification du second type de mouvement, la première sortie étant une affirmation d'un état de crise du sujet et la seconde sortie étant au moins l'une d'une sortie nulle et d'une affirmation d'un état sans crise du sujet ; et
les données de mouvement de sujet étant en outre **caractérisées par** au moins l'une des suivantes :
une première période étant à un minimum et/ou un maximum d'une première plage de périodes pour au moins une portion de la première période de temps,
la première largeur de bande étant à un minimum et/ou un maximum d'une première plage de largeurs de bande pour au moins une portion de la première période de temps,
l'angle décalé étant à un minimum et/ou un maximum d'une plage d'angles décalés pour au moins une portion de la première période de temps,
le premier paramètre de rotation étant à un minimum et/ou un maximum d'une plage de rotations pour au moins une portion de la première période de temps, une seconde période étant à un minimum et/ou un maximum d'une seconde plage de périodes pour au moins une portion de la seconde période de temps, ou
la seconde largeur de bande étant à un minimum et/ou un maximum d'une seconde plage de largeurs de bande pour au moins une portion de la seconde période de temps.

2. Procédé selon la revendication 1,
le premier type de mouvement étant en outre **caractérisé par** :
une première amplitude qui se trouve inclusivement à l'intérieur d'une première plage d'amplitudes, et
le second type de mouvement étant en outre **caractérisé par** :
une seconde amplitude qui se trouve inclusivement à l'intérieur d'une seconde plage d'amplitudes.

3. Procédé selon la revendication 1, le signal étant fourni par un accéléromètre accouplé au sujet et disposé pour acquérir les données de mouvement de sujet.

4. Procédé selon la revendication 3, l'accéléromètre définissant un axe de sujet s'étendant à l'écart du sujet pour définir une orientation de la ligne de base du sujet, la position couchée et l'orientation verticale étant chacune indiquée en référence à l'orientation de la ligne de base.

5. Procédé selon la revendication 1, le signal étant fourni par un dispositif de visualisation qui acquiert une image du sujet, l'image du sujet correspondant aux données de mouvement de sujet.

6. Procédé selon la revendication 5, le dispositif de visualisation acquérant l'image du sujet par l'intermédiaire d'au moins l'un d'un dispositif vidéo ou d'un système d'imagerie thermique et au moins l'un d'un détecteur de mouvement, d'un capteur de profondeur, ou d'un dispositif laser infrarouge.

7. Système de suivi de mouvement pour surveiller un mouvement d'un sujet, le système de suivi de mouvement comprenant :
un logement ;
un système de montage configuré pour accoupler le logement au sujet ;
un accéléromètre disposé sur le logement, l'accéléromètre configuré pour obtenir des données de mouvement de sujet, les données de mouvement de sujet comprenant des données de position de sujet et des données de changement de position du sujet ; et
un processeur configuré pour analyser les données de mouvement de sujet pour effectuer une distinction entre un premier type de mouvement survenant sur une première période de temps et un second type de mouvement survenant sur une seconde période de temps,
le premier type de mouvement étant **caractérisé par** :
une première largeur de bande qui se trouve inclusivement à l'intérieur d'une première plage de largeurs de bande,
les données de position de sujet indiquant que le sujet se trouve dans une orientation couchée à travers la première période de temps, l'orientation couchée définie par un étalonnage initial durant lequel le sujet se trouve en position couchée tout en définissant un angle décalé entre un axe de sujet s'étendant depuis le sujet et un axe vertical, l'orientation couchée définie en outre par l'axe de sujet restant inclusivement à l'intérieur d'une plage d'angles décalés à travers la première période de temps, et
les données de changement de position du sujet indiquant qu'un premier paramètre de rotation des données de changement de position du sujet se trouve inclusivement à l'intérieur d'une plage de rotations à travers la première période de temps, et
le second type de mouvement étant **caractérisé par** :
une seconde largeur de bande qui se trouve inclusivement à l'intérieur d'une seconde plage de largeurs de bande,
les données de position de sujet indiquant que le sujet se trouve dans une orientation verticale à travers la seconde période de temps, l'orientation verticale définie par l'angle décalé qui est égal ou qui excède un seuil d'angle décalé à travers la seconde période de temps, et
les données de changement de position du sujet indiquant qu'un second paramètre de rotation des données de changement de position du sujet est supérieur à un seuil de rotation à travers la seconde période de temps ; et
une interface sensible au processeur, l'interface fournissant une première sortie depuis le processeur en réponse à une identification du premier type de mouvement et fournissant une seconde sortie depuis le processeur en réponse à une identification du second type de mouvement, la première sortie étant une affirmation d'un état de crise du sujet et la seconde sortie étant au moins l'une d'une sortie nulle et d'une affirmation d'un état sans crise du sujet ; et
les données de mouvement de sujet étant en outre **caractérisées par** au moins l'une des suivantes :
une première période étant à un minimum et/ou un maximum d'une première plage de périodes pour au moins une portion de la première période de temps,
la première largeur de bande étant à un minimum et/ou un maximum d'une première plage de largeurs de bande pour au moins une portion de la première période de temps,
l'angle décalé étant à un minimum et/ou un maximum d'une plage d'angles décalés pour au moins une portion de la première période de temps,
le premier paramètre de rotation étant à un minimum et/ou un maximum d'une plage de rotations pour au moins une portion de la première période de temps, une seconde période étant à un minimum et/ou un maximum de la seconde plage de périodes pour au moins une portion de la seconde période de temps, ou
la seconde largeur de bande étant à un minimum et/ou un maximum de la seconde plage de largeurs de bande pour au moins une portion de la seconde période de temps.

8. Système de suivi de mouvement selon la revendication 7,
le premier type de mouvement étant en outre **caractérisé par** :
une première amplitude qui se trouve inclusivement à l'intérieur d'une première plage d'amplitudes, et
le second type de mouvement étant en outre **caractérisé par** :
une seconde amplitude qui se trouve inclusivement à l'intérieur d'une seconde plage d'amplitudes.

9. Système de suivi de mouvement selon la revendication 7, l'accéléromètre définissant un axe de sujet s'étendant à l'écart du sujet pour définir une orientation de la ligne de base du sujet, la position couchée et l'orientation verticale étant chacune indiquée en référence à l'orientation de la ligne de base.
